# EUROPEAN PATENT SPECIFICATION

(11) **EP 1 395 320 B1**
(45) Date of publication and mention of the grant of the patent: **28.06.2006**
(21) Application number: 02778872.8
(22) Date of filing: 14.05.2002
(51) Int. Cl.: A61M 15/00

(54) **MEDICAMENT DISPENSER**
MEDIKAMENTENSPENDER
DISTRIBUTEUR DE MEDICAMENTS

(30) Priority: 11.06.2001 GB 0114176; 11.06.2001 GB 0114175
(43) Date of publication of application: 10.03.2004
(73) Proprietor: GLAXO GROUP LIMITED, Greenford, Middlesex UB6 ONN (GB)
(72) Inventor: WALKER, Richard Ian, Ware, Herts SG12 0DP (GB); BEAUMONT, Gary Robert, Ware, Herts SG12 0DP (GB); JONES, Anthony Patrick, Ware, Herts SG12 0DP (GB); DAVIES, Michael Birsha, Ware, Herts SG12 0DP (GB)
(74) Representative: Rice, Jason Neale
(86) International application number: PCT/EP2002/005320
(87) International publication number: WO 2002/100469

(56) References cited:
- WO-A-00/55072
- WO-A-01/28615
- WO-A-01/41845
- WO-A-01/41846
- US-A- 5 451 105

## Description

This invention relates to a medicament dispenser including a medicament container having a dispensing mechanism actuable by an actuator. The dispenser is particularly suitable for use as an inhalation device.

It is well known to treat patients with medicaments contained in an aerosol, for example, in the treatment of respiratory disorders. It is also known to use for such treatment, medicaments which are contained in an aerosol and are administered to a patient by means of an inhalation device comprising a tubular housing or sleeve in which the aerosol container is located and an outlet tube leading out of the tubular housing. Such inhalation devices are generally referred to as metered dose inhalers (MDIs). The aerosol containers used in such inhalation devices are designed to deliver a predetermined dose of medicament upon each actuation by means of an outlet valve member at one end which can be opened either by depressing the valve member while the container is held stationary or by depressing the container while the valve member is held stationary. In the use of such devices, the aerosol container is placed in the tubular housing with the outlet valve member of the container communicating via a support with the outlet tube, for example a nozzle or mouthpiece. When used for dispensing medicaments, for example in bronchodilation therapy, the patient then holds the housing in a more or less upright condition and the mouthpiece or nozzle of the inhalation device is placed in the mouth or nose of the patient. The aerosol container is pressed towards the support to dispense a dose of medicament from the container which is then inhaled by the patient.

It is also known to use dry powder inhalation devices for the delivery of inhalable medicament. In one aspect, such dispensers comprise pre-metered doses of powdered medicament, for example in capsules or blisters. In another aspect, such dispensers comprise a reservoir of powdered medicament from which doses are metered prior to or concurrent with the delivery process. In either case, the device may be designed for passive release of medicament, where the medicament is simply made available at a delivery position for aerosolisation in response to the inhalation of the patient. Alternatively, an active release mechanism may be used whereby a 'puff' of gas or air is provided to the delivery position to assist in aerosolisation of the powder prior to or concurrent with the inhalation of the patient. Such devices are generally called active release dry powder inhalers (active DPIs). The source of the compressed gas or air is generally an aerosol container but can also be provided by another suitable means such as a pump or plunger mechanism.

It is also well known to use syringes for the delivery of injectable medicament to a patient. Traditional syringes rely on puncturing of the patient's skin by a hollow needle through which the injectable medicament (in solution or suspension form) is delivered to the muscle or tissue of the patient. Recently developed needleless systems for the delivery of injectables employ high velocity injection of particle formulated drugs or vaccine through the skin and into any physically accessible tissue. Other needleless systems employ similar high velocity injection of drug or vaccine coated on to a suitable carrier particle. Such needleless systems may be configured to include a source of compressed air or gas, which on release provides energy to propel the medicament particles for injection into the skin.

It may be understood that effective delivery of medicament to the patient using an inhalation device such as an MDI or active DPI as described above is to an extent dependent on the patient's ability to manually actuate the device (e.g. firing of the aerosol) and to co-ordinate the actuation thereof with the taking of a sufficiently strong inward breath. For some patients, particularly young children, the elderly and the arthritic, manual actuation of the device can present difficulties. Other patients find it difficult to co-ordinate the taking of a reliable inward breath with actuation of the device. Both of these sets of patients run the risk that they do not receive the appropriate dose of medicament.

It may also be understood that effective delivery of medicament to the patient using a syringe or needleless injection system as described above also requires care and dexterity.

Commonly owned International (PCT) Application WO-A-01/41849 Published 14.06.2001 discloses a medicament dispenser which does not require controlled manual actuation by the patient. In one aspect, the dispenser comprises a medicament container having a dispensing mechanism such as a valve or plunger and an actuator for actuating the dispensing mechanism. In another aspect, the device comprises an active DPI or needless injection system having a source of compressed air or gas having a dispensing mechanism such as a valve or plunger and an actuator for actuating the dispensing mechanism. Actuation is responsive to the application of non-mechanical (e.g. electrical) energy to drive means of the actuator. The non-mechanical energy can in turn be provided in response to the sensing of the breath of a patient.

The Applicant has now developed improvements to, and variations on, the dispenser disclosed in the aforementioned PCT application. The improvement comprises adjusting means adapted to enable medicament containers of varying sizes to be accepted with the housing.

As further background prior art there may be mentioned WO-A-00/55072, WO-A-01/28615, US-A-5451105, WO-A-01/41845 and WO-A-01/41846.

According to one aspect of the present invention there is provided a medicament dispensers according to claim 1.

Suitably, the medicament container comprises a release mechanism for releasing medicament therefrom, such as a valve or other suitable mechanism which may also provide a metering function. In another aspect, the medicament container may be arranged for rupture in response to firing of the aerosol container, thereby making the medicament contents thereof available for energisation. In one aspect, the medicamerit dispenser is in the form of an active DPI in which a 'puff' of air or gas (e.g. helium) is delivered from an aerosol container, pump or plunger mechanism to aerosolise a dose of released dry powder medicament. In another aspect, the medicament dispenser is in the form of a needless injection system in which compressed air or gas (e.g. helium) is delivered at high velocity from the aerosol container to propel a dose of dry powder medicament for injection into the skin. Thus, suitably the aerosol container, which as used herein refers to any suitable container for comprising liquefied gas under pressure, comprises a compressed air or gas (e.g. helium).

The release mechanism comprises means for the making medicament available from the medicament container for release to the patient. The release may be active in the sense that medicament is actively dispensed from the container, or the release may be passive in the sense that medicament is merely made available for release when the release means is actuated. Release may be to a delivery position for delivery to the patient, or alternatively there may be a further transport step in which the medicament is transported from a non-delivery, position, to a position where the drug is ready for delivery to the patient

In one aspect, the medicament is pre-metered prior to release. For example, the medicament is pre-metered in capsules, strip or tape form.

In another aspect, the medicament container comprises a reservoir for medicament (e.g. in powder form) and a meter is provided for metering an amount (e.g. by volume or by weight) of medicament from said reservoir.

Suitably, the adjusting means comprises variable engaging means. In one aspect, the variable engaging means comprises a first engager located on a first part of the medicament dispenser and a second engager located on a second part of the medicament dispenser, wherein the first and second engager are engageable with the medicament container (e.g. at a variety of positions).

In a first embodiment, the adjusting means comprises a clutch and drive plate mechanism which interconnects with a carriage shaft which houses the medicament container. A coupling mechanism is connected to the drive plate. The clutch permits the shaft to find its rest position without applying any load. Once the coupling mechanism is activated, the clutch grips the shaft and the drive plate is pulled down.

In a second embodiment, the adjusting means comprises a rack and wheel mechanism. A medicament container assembly is provided with a plurality of wheels which are adapted to mesh with a rack located on the inside of a housing. As the device is activated, the wheels mesh with the rack on the body. The wheels lock enabling the can assembly to move with the body causing the can to fire. After firing, activation is stopped, the wires begin to relax and the body and can assembly return to their resting position. Suitably, the medicament container assembly is further provided with a collar having a rack profile with which the wheels can also mesh during activation. Suitably, activation is by means of a coupling device.

In a third embodiment, the adjusting means comprises a flexible bag containing a viscous fluid, suitably located in the base of the housing. When the medicament (or aerosol) container is received by the housing, the flexible bag forms around the can to provide a zero datum point

In a fourth embodiment, the adjusting means comprises a drive wedge, the drive wedge having wedge shaped teeth, which cooperates with toothed carriage adapted to accommodate the medicament container. Suitably, the teeth on the carriage are of a truncated wedge shape. Suitably, the number of teeth on the carriage equals the number of teeth on the drive wedge. The wedge shaped teeth on the drive wedge allows the carriage to engage at varying positions depending on the height of the medicament container used. Suitably, a drive plate is in contact with the drive wedge. Suitably, one or more coupling mechanisms are attached to the drive plate at one end and to the far end of the carriage at the other end. As the carriage is rotated from a load position to the engage position, the teeth on the carriage engage with the wedge shaped teeth on the drive wedge. As the carriage is rotated further, the medicament container is moved into the dispensing position and will fire. Once fired the carriage is rotated back to the rest position.

Suitably, the dispensing mechanism is selected from the group consisting of a valve, pump or plunger mechanism. Preferably, the dispensing mechanism comprises a metering mechanism.

In one aspect, the dispensing mechanism comprises a valve. Suitably, the valve is a slide valve. Other valve systems include, but are not limited to, poppet valve systems, wedge gate valve systems, double-disc gate valve systems, globe and angle valve systems, swing check valve systems, end cock valve systems, and other like valve systems. The valve design is typically a function of providing a predetermined dosage or amount of the medicament contained within the container to a user.

Where the medicament container is a pressurized aerosol container, the valve typically comprises a valve body having an inlet port through which a medicament aerosol formulation may enter said valve body, an outlet port through which the aerosol may exit the valve body and an open/close mechanism by means of which flow through said outlet port is controllable.

The valve may be a slide valve wherein the open/close mechanism comprises a sealing ring and receivable by the sealing ring a valve stem having a dispensing passage, the valve stem being slidably movable within the ring from a valve-dosed to a valve-open position in which the interior of the valve body is in communication with the exterior of the valve body via the dispensing passage.

Typically, the valve is a metering valve. The metering volumes are typically from 10 to 100 µl, such as 25 µl, 50 µl or 63 µl. Suitably, the valve body defines a metering chamber for metering an amount of medicament formulation and an open/close mechanism by means of which the flow through the inlet port to the metering chamber is controllable. Preferably, the valve body has a sampling chamber in communication with the metering chamber via a second inlet port, said inlet port being controllable by means of an open/close mechanism thereby regulating the flow of medicament formulation into the metering chamber.

The valve may also comprise a 'free flow aerosol valve' having a chamber and a valve stem extending into the chamber and movable relative to the chamber between dispensing and non-dispensing positions. The valve stem has a configuration and the chamber has an internal configuration such that a metered volume is defined therebetween and such that during movement between is non-dispensing and dispensing positions the valve stem sequentially: (i) allows free flow of aerosol formulation into the chamber, (ii) defines a closed metered volume for pressurized aerosol formulation between the external surface of the valve stem and internal surface of the chamber, and (iii) moves with the dosed metered volume within the chamber without decreasing the volume of the closed metered volume until the metered volume communicates with an outlet passage thereby allowing dispensing of the metered volume of pressurized aerosol formulation. A valve of this type is described in U.S. Patent No. 5,772,085.

The valve may also have a structure and action similar to those aerosol valves described in European Patent Application No. EP-A-870,699 and PCT Patent Application No. WO99/36334.

In another aspect, the dispensing mechanism comprises a plunger, such as might exist in a syringe for the delivery of injectable medicament to a patient. Embodiments including multiple plungers and multiple syringe chambers are also envisaged. The syringe conterits may for example, be liquid, solutions, suspensions, particulates or in freeze-dried form. A retract or reset mechanism is typically provided for the plunger.

Traditional syringes rely on puncturing of the patient's skin by a hollow needle through which the injectable medicament (in solution or suspension form) is delivered to the muscle or tissue of the patient. Recently developed needleless systems for the delivery of injectables employ high velocity injection of particle formulated drugs or vaccine through the skin and into any physically accessible tissue. Other needleless systems employ similar high velocity injection of drug or vaccine coated on to a suitable carrier particle.

In another aspect, the dispensing mechanism comprises a pump mechanism such as might be found in a dispenser for dispensing liquid or solution (e.g. aqueous solution) form medicament. The pump may deliver the medicament directly to the patient (e.g. as a nasal spray) or the pump may deliver the medicament to an intermediate position at which further energy is supplied thereto to further propel, aerosolise or otherwise direct the medicament dose to the patient

A reset mechanism may be provided for resetting the dispensing mechanism after actuation thereof. The reset mechanism may for example, comprise a spring, motor, mechanical arrangement or a reset coupling. The reset mechanism may in aspects, form part of the dispensing mechanism or part of the drive means. Alternatively, it may be an entirely separate mechanism.

The term 'drive means' is used herein to define any means capable of providing the required drive action. The drive suitably allows for movement to reverse the drive action. Thus, it suitably allows for both actuation and resetting of the dispensing mechanism. Whilst, the drive action is almost always powered, the return action may be powered or it may be a simple, unpowered relax action or a combination of these alternatives.

In aspects, the drive means is used in combination with a gear mechanism. The gear mechanism enhances the torque (force) provided by the drive means, which thereby enables relatively compact, low energy and low torque producing motors to be employed. Compactness is a key requirement of portable medicament dispensers, particularly those designed for hand-held use and/or pocket portability. The Applicants have also found that enhanced utility can be obtained if electronic control systems are provided to control the operation of the drive means.

The drive means may also be configured to agitate (e.g. vibrate or shake) the medicament container at any point during the drive action.

The drive means typically comprises a motor, preferably an electrically-powered motor. The motor may provide linear or rotary drive, but linear motors are most suitable. The motor may for example, comprise a DC electric motor, a piezoelectric (PZ) motor, a solenoid motor or a linear motor.

The drive means may also comprise a fluid pump such as an air pump or hydraulic pump.

The drive means may have direct driving action such that actuation thereof results directly in actuation of the dispensing mechanism. Alternatively, the drive means may be coupled through some sort of coupling to the container seat/dispenser seat. This coupling may be a direct coupling or it may comprise some sort of energy storage mechanism or trigger mechanism. Embodiments are for example, envisaged in which the drive means is used to provide energy to a sprung trigger system, the actuation of which results in movement of the container seat/dispenser seat to actuate the dispensing mechanism.

The comprising of the drive means may deform, or undergoes a phase transition; to result in a change in shape/dimension of the coupling which serves to actuate the dispensing mechanism.

Preferably, the electrical current energy comprises electric current flow through the coupling or reset coupling.

Preferably, the coupling or any reset coupling comprises a wire, strip, coil or tube.

Arrangements comprising multiple strips, wires, coils, or tubes are also envisaged. The multiple strips, wires, coils, or tubes may be arranged in any suitable fashion including parallel or series arrangements and bundle arrangements.

The coupling may be coated with any suitable coating, or encased within any suitable encasing including a shrink-wrap sheath.

In one particular aspect, the coupling comprises one or more wires which contract in response to application of non-mechanical energy thereto.

Preferably, the degree of contraction of the coupling is from 2% to 8%.

In embodiments, the coupling comprises an alloy which undergoes a phase transition on heating (shape memory alloys). Certain shape memory alloys also undergo a change in shape on recooling without externally applied energy. Such two way shape memory alloys are also envisaged for use herein.

In one embodiment, the shape memory alloy is a binary alloy, preferably a nickel-titanium alloy such as a nickel-titanium alloy comprising from 5% to 95%, preferably from 20% to 80%, nickel by weight and from 95% to 5%, preferably from 80% to 20%, titanium by weight. By nickel-titanium alloy it is meant an alloy comprised essentially of nickel and titanium, although other elements such as Cu and Nb may be present in small (e.g. trace) amounts.

In other embodiments, the shape memory alloy is a tertiary alloy, preferably a copper-aluminium-nickel alloy or a copper-zinc-aluminium alloy. Trace amounts of other elements may also be present.

In further embodiments, the coupling comprises an alloy which undergoes a phase transition on application of a magnetic field thereto (magnetic shape memory alloys). These materials are generally intermetallic, ferromagnetic alloys that exhibit twin variants in the martensitic, or low-temperature, phase of the material. Suitable magnetic shape memory alloys are for example, described in US Patent No. 5,958,154.

In one embodiment, the magnetic shape memory alloy exhibits an austenitic crystal structure above a characteristic phase transformation temperature and also exhibits a martensitic twinned crystal structure below the phase transformation temperature. The alloy has a magnetocrystalline anisotropy energy that is sufficient to enable motion of twin boundaries of the martensitic twinned crystal structure in response to application of a magnetic field to the martensitic twinned crystal structure.

Where a magnetic shape memory alloy is employed the medicament dispenser preferably includes a magnetic field source disposed with respect to the coupling in an orientation that applies to the coupling a magnetic actuation field in a direction that is substantially parallel with a selected twin boundary direction of the martensitic twinned crystal structure of the coupling material.

Alternatively, the medicament dispenser preferably includes a magnetic bias field source disposed with respect to the coupling in an orientation that applies a magnetic bias field to the coupling, and a magnetic actuation field source disposed with respect to the coupling in an orientation that applies a magnetic actuation field to the coupling material in a direction that is substantially perpendicular to the orientation of the applied magnetic bias field.

A preferred magnetic shape memory alloy is the actuator material comprising an alloy composition defined as Ni_{65-x-y}Mn₂₀+xGa₁₅+y, where x is between 3 atomic % and 15 atomic % and y is between 3 atomic % and 12 atomic %. Preferably, the actuator material comprises an alloy composition defined as Ni_{65-x-y}Mn₂₀+xGa₁₅+y, where x is between 6 atomic % and 10 atomic % and y is between 5 atomic % and 9 atomic %; or where x is between 12 atomic % and 15 atomic % and y is between 3 atomic % and 6 atomic %; or where x is between 10 atomic % and 14 atomic % and y is between 3 atomic % and 6 atomic %; or where x is between 7 atomic % and 11 atomic % and y is between 3 atomic % and 7 atomic %. In a particularly preferred aspect, the alloy is Ni₅₀Mn₂₆Ga₂₅.

Another preferred magnetic shape memory alloy is the alloy having the composition (NiₐFe_{b}Co_{c})_{85-x-y}(Mn_{d}FeₑCo_{f})₂₀+x(Ga_{g}SiₕAlᵢ)₁₅+y, where x is between 3 atomic % and 15 atomic % and y is between 3 atomic % and 12 atomic %, and where a+b+c=1, where d+e+f=1, and g+h+i=1.

In preferred aspects, b is between zero and 0.6, c is between zero and 0.6, and e, f, h and i are each zero; or b and c are each zero, e is between zero and 0.6, f is between zero and 0.6, and h and i are each zero; or b, c, e and f are each zero, h is between zero and 0.5, and i is between zero and 0.5.

Other suitable shape memory alloys include those based on ion-exchange polymer composites such as are described in 'Ionic Polymer-Metal Composites (IPMC) As Biomimetic Sensors, Actuators & Artificial Muscles - A Review', M.

Shahinpoor, Y. Bar-Cohen, J. O. Simpson and J. Smith as published at http://www.unm.edu/-amri/paper.html.

Other potentially suitable shape memory alloys include those based on contractile polymers such as are described in 'Review of Artificial Muscle based on Contractile Polymers', Massachusetts Institute of Technology Artificial Intelligence Laboratory Memo No. 1330, November 1991, David L. Brock.

Preferably, the one or more wires have a diameter from 30 to 400 micrometers, preferably from 50 to 150 micrometers.

Preferably, the coupling comprises from two to twenty, preferably six to twelve wires which contract in response to the application of non-mechanical energy thereto. The wires may be arranged in any suitable fashion including parallel or series arrangements and bundle arrangements.

In another aspect, the coupling comprises a strip which comprises multiple layers of different metals. Suitable strips typically comprise a plurality of layers of material, each material having a different coefficient of thermal expansion.

Preferred examples of strips indude those comprising multiple layers of different metals (e.g. bimetallic strips) and strips comprising at least one piezoelectric material. Suitable piezoelectric materials include piezoelectric ceramics, such as compounds of lead zirconate and lead titanate, and piezoelectric crystals which are generally polycrystalline ferroelectric materials with the perovskite structure. Such piezoelectric materials generally deform in response to the application of an electric field.

In one aspect, the coupling is deformable in response to heating arising from electrical current flow in the range from 0.01A to 100A, preferably from 0.1A to 5A.

In another aspect, the coupling is deformable in response to the application of an electrical field, particularly where the coupling comprises a piezoelectric material.

In a further aspect, the coupling is deformable in response to a magnetic field of from 0.01 to 100 Tesla. The magnetic field may for example, be produced by a permanent magnet or by an electromagnet.

In a preferred aspect, the drive means additionally comprises gear means to gear up the force (torque) provided thereby. This enables motors of relatively low torque (which are typically compact, high speed and with low power requirements) to be employed.

The Applicants have determined that gear ratios of from 20:1 to 1000:1, particularly from 50:1 to 500:1, preferably 100:1 to 200:1 are most suitable.

In a particular example, the force required to actuate the valve of a typically metered dose inhaler (MDI) dispenser is from 20 to 40N. But if a gear means is employed motors capable of providing torque of less than 1N.m may be employed.

The gear means may comprise any suitable gear mechanism. Specific examples include cam gears, screw drives, levers, crank shafts, pulleys and hydraulic gears. Preferably, the gear mechanism is relatively compact.

The medicament dispenser of the invention is configured for use with medicament containers of varying sizes, shapes and/or manufacturing tolerances. It is therefore desirable that the medicament dispenser herein comprises locator means to locate the position of the medicament container. The locator may also act such as to continually track the position of the medicament container e.g. during any particular drive operation. The medicament dispenser also preferably comprises zeroing means to position the medicament container at a defined zero position therein.

The locator, tracker and/or zeroing means may comprise mechanical and/or electronic components such as any known electronic position sensing means.

Suitably, the drive means is actuable in response to electrical current flow in the range from 0.01A to 100A, preferably from 0.1A to 5A.

In a further improvement aspect, the medicament dispenser is provided with decoupling means adapted to enable the medicament container to return to a non-dispensing position (deactivation of the dispensing mechanism) independently to resetting of the drive (e.g. coupling) mechanism. Once, the medicament container is returned to the non-dispensing position it may be refilled. There are two advantages to enabling the medicament container to be refilled prior to complete resetting of the coupling mechanism: firstly, multiple doses may need to be given in quick succession, and secondly, to ensure the correct concentration of active component is placed in the medicament container.

Suitably, the decoupling means is movable from a first non-dispensing position through a dispensing position to a second non-dispensing position. The second non-dispensing position may be the same as or different to the first non-dispensing position.

Suitably, movement of the decoupling means in one direction, is translated into movement of the medicament (or aerosol) container between dispensing position and non-dispensing positions.

Suitably, the decoupling means comprises a gearing mechanism, capable of moving the medicament container from a non-dispensing position, through a dispensing position and back to a non-dispensing position. The gearing mechanism may comprise gears and/or levers.

A first embodiment of this 'decoupling' improvement aspect provides decoupling means having a multi-sided or multi-lobed cam in contact either directly or indirectly with a medicament container, and a coupling mechanism extending from the cam to a fixed point on a medicament dispenser. Preferably, the cam has at its centre a clutch which will enable any slack in the wires after relaxation to be taken up. As the coupling mechanism is activated the cam rotates and exerts a downward pressure on the medicament container, moving it into a dispensing position. Once the medicament is dispensed, the cam quickly rotates further back to a resting position, the medicament container moving back to the non-dispensing position.

A second embodiment of this 'decoupling' improvement aspect provides decoupling means comprising a beam, rotatably hinged at its centre point, said beam being movable between a first non-dispensing position through a dispensing position to a second non-dispensing position by activation of a coupling mechanism. Suitably, a first end of the beam is attached to a fixed point, for example on the housing and the second end of the beam attached to the medicament container, such as a dispensing can. Upon activation of the coupling mechanism, the beam is moved from its first non-dispensing position through the dispensing position to its second non-dispensing position. When the dispensing position is reached, the active ingredient is fired from the medicament container. As the beam passes the dispensing position, it quickly proceeds to the second non-dispensing position, suitably with the aid of a recovery valve spring. The coupling mechanism used to move the beam from its first position to its second position is suitably two or more wires attached to opposite sides of the housing.

A third embodiment of this 'decoupling' improvement aspect provides decoupling means comprising a top crown and a push crown, the two crowns engaging with each other. The push crown is in contact with a medicament container. Activation of a coupling mechanism, for example coupling wires causes the top crown to rotate which in turn exerts a downward force on the medicament container through the top crown, moving the medicament container to a dispensing position. Suitably, the two crowns use a tooth/tooth or ball/tooth arrangement to engage. Suitably, a dutch is provided to enable the coupling mechanism to return to the rest position.

In a further improvement aspect, the improvement comprises drive-assisting means adapted to provide a mechanical advantage to the drive mechanism. The drive-assisting means may act at the same time as or sequentially with activation of the drive mechanism. For example, increased movement of the dispensing mechanism, such as a valve or plunger, with respect to the decrease in the length of a wire coupling mechanism may be obtained. In SMA wire-driven aspects, the decrease in the length of the wire is only a few percent of the total length of the wire, for example 2 to 8 %. Accordingly, in order for the dispensing mechanism to be moved sufficiently for the drug to be dispensed, the length of wire required would be considerable and in many cases impractical. By incorporating the drive-assisting (i.e. wire-assisting) the small decrease in the length of the wire on application of non-mechanical energy can be translated into a larger movement of the dispensing mechanism. Alternatively, a change in the work profile of the coupling mechanism may be obtained.

Suitably, the drive-assisting means provides a mechanical advantage on activation of the drive mechanism. Suitably, the mechanical advantage is to provide increased movement of the dispensing mechanism with respect to movement of the drive mechanism. Alternatively, the mechanical advantage is to provide an increased force in the stroke of the dispensing mechanism.

Suitably, the drive-assissting means comprises a gearing mechanism linking the drive mechanism and the dispensing mechanism. Suitably, the gearing mechanism comprises gears and/or levers.

In a first embodiment of the 'drive-assist' improvement, the drive-assisting means comprises one or more levers and one or more pivot points wherein one or more drive mechanisms are attached to at least one lever and wherein at least one of said one or more levers are in contact with a medicament container. As the drive mechanism is activated, for example as the wires contract, the lever and pivot arrangement is moved such that a downward force is exerted on a medicament container, moving the medicament container into a dispensing position. A number of alternative arrangements may be envisaged which would provide the required result, a number of which will be described in more detail hereinafter. However, the present invention is not restricted to these particular arrangements and all arrangements are within the scope of the invention.

In a second embodiment of the 'drive-assist' improvement, the drive-assisting means comprises a rotatable cylinder comprising a downwardly spiralling guide track, a guide arm fixed at the lower end to a medicament container, wherein the guide arm is locatable within the guide track, and one or more drive mechanisms connected at one end to the rotatable cylinder. Preferably the rotatable cylinder also comprises a clutch to assist the drive mechanisms to return to the rest position. When the drive mechanism(s) moves, the cylinder begins to rotate causing the guide arm to move downwards in a vertical manner, and causing the medicament container to move to a dispensing position. Preferably, a spring is provided between the bottom of the cylinder and the can. The spring is provided to take up any float between the guide arm and guide track. Once the medicament has been dispensed, the force of the compressed valve spring returns the medicament container and drive-assisting means to its rest position.

In a third embodiment of the 'drive-assist' improvement aspect, the drive-assisting means comprises a bowed beam connected at each end to a coupling mechanism, the beam having a notch removed in the lower surface thereof. Suitably, the notch is centrally located in the lower surface of the beam. The beam rests at either end on two vertical carriages, which in turn are connected to the dispensing can. As the drive moves, both ends of the beam are pulled downwards until the beam is straight and the notch closed which in turn forces the two vertical carriages to move the dispensing can into a dispensing position. As the drive again relaxes, the beam rebends and the dispensing can moves upwards into the non-dispensing position.

In a fourth embodiment of the 'drive-assist' improvement aspect, the drive-assisting means comprises an arrangement, adapted to change the work profile of the drive mechanism to activate the dispensing mechanism. Initially, a high force is provided against which the drive will work. As actuation of the drive continues, the force switches so that it works with the wires. Such an arrangement may be provided by a spring and lever arrangement or by for example an spring steel plate.

In a further improvement aspect, the improvement: comprises providing a medicament dispenser which has a use configuration and a storage configuration wherein the medicament dispenser in its storage configuration is smaller than when in the use configuration.

Suitably, the housing comprises a telescopic arrangement, for example concentric cylinders which when in storage configuration nest inside one another, but extend when in use. Suitably, the concentric cylinders lock to one another when extended. The drive mechanism, for example a coupling wire mechanism such as an SMA wire, is suitably attached to the outermost concentric cylinder; the coupling mechanism is slack when the device is in the storage configuration but is held taut when the device is extended to the use configuration. The outermost cylinder having the coupling device attached thereto may be of a double-walled construction enclosed at the top, the coupling device being encompassed between the two walls for additional protection. The outer wall may be longer than the inner wall.

In a further improvement aspect, there is provided a disposable medicament container assembly for incorporation into a medicament dispenser, comprising a medicament container having a stem, a top cap, a stem drive adapted to accept the stem through an opening in the centre thereof, wherein a drive mechanism is attached from the top cap to the stem drive. Suitably, the stem drive further has an outlet port through which the medicament can be dispensed. Suitably a number of drive coupling mechanisms extend from the top cap to the stem drive; suitably the coupling mechanisms are wires, for example shape memory alloy wires. Suitably, the medicament container is provided with a sleeve, which is suitably electrically and/or thermally insulating. Suitably, the medicament container and top cap has a spacer located therebetween.

Such a disposable medicament container assembly would have many advantages, for example any tolerance issues due to variations in medicament container size would be overcome and fatigue of the coupling mechanism would be reduced as the coupling mechanism would only need to last for the number of doses contained in the medicament container.

Preferably, the medicament dispenser additionally comprises an electrical energy source for providing electric current, or for providing an electric field, or for powering an electromagnet to provide a magnetic field. In one aspect, the electrical energy source comprises a voltaic cell or battery of voltaic cells which may be rechargeable. In another aspect, the electrical energy source comprises a photovoltaic cell or battery of photovoltaic cells. In a further aspect, the electrical energy source comprises a converter for converting mechanical energy into electrical energy. In a further aspect, the electrical energy source comprises a capacitor for local storage of charge. Suitable capacitors comprise those known as 'super capacitors' with a high capacitance to size ratio, such as those consisting of solid electrodes and liquid electrolyte.

Any known systems for power management and conservation may be employed with the electrical energy source to manage and/or conserve the power output thereof. Energy may be conserved by a variety of means to enable the device to operate for longer on a given source of energy, such as a battery. Energy conservation or saving methods have additional advantages in terms of reducing the size requirements of the power source (e.g. battery) and thus the weight and portability of the medicament dispenser.

A variety of energy saving methods are available which generally involve reducing power consumption. One such method is to use a clock or timer circuit to switch the power on and off at regular or predetermined intervals. In another method the system can selectively switch on/off specific electronic devices, such as visual display units or sensors, in order to power these devices only when they are required to perform a particular sequence of events. Thus different electronic devices may be switched on and off at varying intervals and for varying periods under control of the system. The power sequencing system may also respond to a sensor, such as a motion or breath sensor, which is activated on use of the device.

Low power or "micropower" components should be used within the electronics where possible and if a high power device is required for a particular function this should be put into a low power standby mode or switched off when not required. Similar considerations apply in the selection of transducers. Operation at low voltage is desirable since power dissipation generally increases with voltage.

For low power digital applications complementary metal oxide semi-conductor (CMOS) devices are generally preferred and these may be specially selected by screening for low quiescent currents. Clock speeds of processors and other logic circuits should be reduced to the minimum required for computational throughput as power consumption increases with frequency. Supply voltages should also be kept at minimal values consistent with reliable operation because power dissipation in charging internal capacitance's during switching is proportional to the square of the voltage. Where possible, supply voltages should be approximately the same throughout the circuit to prevent current flowing through input protection circuits. Logic inputs should not be left floating and circuits should be arranged so that power consumption is minimised in the most usual logic output state. Slow logic transitions are undesirable because they can result in relatively large class-A currents flowing. Resistors may be incorporated in the power supply to individual devices in order to minimise current in the event of failure.

In some control applications, devices that switch between on and off states are preferred to those that allow analog (e.g. linear) control because less power is dissipated in low resistance on states and low current off states. Where linear components are used (e.g. certain types of voltage regulators) then types with low quiescent currents should be selected. In some circuit configurations it is preferable to use appropriate reactive components (i.e. inductors and capacitors) to reduce power dissipation in resistive components.

Any electrical circuit may incorporate voltage amplification means for generating a higher voltage than that supplied by the voltaic cell or battery of voltaic cells, for example a step-up or inverting switching circuit or a dc-dc converter incorporating an oscillator, transformer and rectifier.

The electrical circuit may incorporate one or more energy storage components such as capacitors or inductors in order to supply a high enough instantaneous current to raise the temperature of the strips or wires at the required rate to the required temperature.

The input to the electrical circuit may be connected to the electrical energy source by means of a mechanical, electro-mechanical or electronic switching component.

The output of the electrical circuit may be connected to the strips or wires or to an electromagnet by means of a mechanical, electro-mechanical or electronic switching component or by a component allowing the output current to be controlled in a linear or digital (e.g. pulse width modulated) manner.

Suitable control profiles (e.g. via pulse width modulation) include those where the temperature of a shape memory alloy coupling is initially raised to a holding temperature (H) which is just below the transition temperature (T). Actuation of the coupling is then achievable by heating the coupling to a temperature (A) just above the transition temperature. This can be achieved rapidly because the holding temperature (H) is close to the transition temperature (T). When the source of heating is switched off, deactuation also occurs rapidly because the cooling from a temperature (A) only just above the transition temperature (T) to the transition temperature involves only a small temperature decrease.

The strip or wire components may be powered from the battery using a switching component without additional power supply circuitry.

Suitably, the medicament dispenser additionally comprises a controller for controlling the amount of electrical current flow through the coupling or to an electromagnet.

Suitably, the medicament dispenser additionally comprlses a timer for controlling the duration of electrical current flow through the coupling or to an electromagnet.

Suitably, the medicament dispenser additionally comprises a local electrical store such as a capacitor or inductor.

Suitably, the medicament dispenser is provided with a manual override to enable actuation of the device in the event of loss of electrical power.

Suitably, the medicament dispenser is provided with child-resistance features to prevent undesirable actuation thereof by a young child.

In one aspect, the container is an aerosol container, preferably comprising a metering valve at the dispensing outlet. In another aspect, the container is for medicament in solution form (e.g aqueous solution) comprising a pump dispensing mechanism.

In one aspect, the aerosol container comprises a suspension of a medicament in a propellant. The propellant preferably comprises liquefied HFA134a, HFA-227, helium or carbon dioxide.

In another aspect, the aerosol container comprises a solution of a medicament in a solvent

The medicament is preferably selected from the group consisting of albuterol, salmeterol, fluticasone propionate, fluticasone furoate, beclomethasone dipropionate, salts or solvates thereof and any mixtures thereof. Alternatively, the dispenser may be employed for dispensing vaccine.

Preferably, the valve is a slide valve.

Preferably, the valve is a metering valve. In alternative embodiments, metering of medicament dose may be achievable by pulsing electrical current flow through the coupling or to the electromagnet for a selected dispensing time.

Preferably, the container seat is shaped for snug receipt of the base of the medicament container or aerosol container. Preferably, the dispenser seat is shaped for snug receipt of the tip of the dispensing mechanism. More preferably, the dispenser seat is further shaped to support the walls of the medicament container or aerosol container. Most preferably, the container seat and dispenser seat in combination form a cradle for the medicament container or aerosol container.

Preferably, the container seat and dispenser seat comprise electrically conducting material and the container is electrically insulated therefrom.

In one aspect, the dispensing mechanism comprises an electrically insulating material.

In another aspect, the dispensing mechanism comprises an electrically conducting material and an insulator is provided between the dispensing mechanism and the dispenser seat.

Preferably, deformation of the coupling and hence, actuation of the dispensing mechanism is responsive to a patient-actuable trigger.

### In one aspect, said trigger comprises a button, switch or lever arrangement

In another aspect, the medicament dispenser is in the form of an inhaler for the delivery of inhalable medicament Inhalation may be through the nose or the mouth. Preferably, actuation of the drive mechanism and hence, actuation of the dispensing mechanism is responsive to a patient-actuable trigger comprising a sensor which senses the breath of a patient. The actuation of the drive mechanism (e.g. by electrical current flow therethrough) may be responsive to the detection of the inward breath of a patient. Alternatively, actuation of the drive mechanism (e.g. by electrical current flow therethrough) may be responsive to a trigger coupled to any point in the breathing pattern of the patient, such as the end of the outward breath.

In one aspect, the sensor comprises a breath-movable element which is movable in response to the breath of a patient. Preferably, the breath-movable element is selected from the group consisting of a vane, a sail, a piston, a diaphragm and an impeller.

Movement of the breath-movable element may be detectable by any suitable technique for detecting movement. Suitable techniques include optical detectors, magnetic detectors or detectors using detection of capacitative effects.

Optical detectors may be used to detect movement of the breath-movable element by providing the element with a patterned outer surface, for example strips in a barcode type arrangement, and locating the optical detector so that it points towards the patterned surface. Movement of the breath-movable element alters the amount of the light source which reflects back onto the optical detector as the beam passes over the patterned surface. The strips may be arranged so that the direction of movement of the element can be detected.

Magnetic detectors may be used to detect the movement of breath-movable element by the use of a magnetic switch device. A reader is located on the dispenser and magnetic material embedded within the breath-movable element (or vice-versa). Movement of the breath-movable element results in a change of the magnetic field experienced by the reader. Alternatively, a Hall effect device can be used whereby a semiconductor measures the strength of the magnetic field of the magnetic material on the breath-movable element.

Detection of capacitative effects may be used to detect movement of the breath-movable element by adding a conductive part to the element and also to a second fixed part of the dispenser. Movement of the breath-movable element results in a change in capacitance which can be measured.

In another aspect, the sensor comprises a pressure sensor for sensing the pressure profile associated with the breath of a patient. A pressure transducer is an example of a suitable pressure sensor.

In another aspect, the sensor comprises an airflow sensor for sensing the airflow profile associated with the breath of a patient

In another aspect, the sensor comprises a temperature sensor for sensing the temperature profile associated with the breath of a patient.

In another aspect, the sensor comprises a moisture sensor for sensing the moisture profile associated with the breath of a patient.

In another aspect, the sensor comprises a gas sensor for sensing the oxygen or carbon dioxide profile associated with the breath of a patient. The chemical profile of the inhaled and exhaled part of the breath cycle varies and this further may be used as a measurement tool.

Suitably, the breath data indudes breath cycle data, FEV₁ and/or peak flow data.

In one aspect, the coupling is exposable to the airflow arising from the inhalation or expiration of the patient to assist in the cooling of the coupling post-actuation of the dispensing mechanism. Other active cooling mechanisms may be employed, such as fan cooling.

Preferably the medicament dispenser comprises an actuation or dose counter for counting the number of actuations of the dispensing mechanism or releases of dose therefrom. The actuation or dose counter may be mechanical or electronic. More preferably the actuation or dose counter is independent of the coupling so that counting will occur even if the dispensing mechanism is manually actuated.

Preferably a manual override is provided to enable manual actuation of the dispensing mechanism. The manual override may be designed to cover all situations in which the coupling does not actuate in the normal manner. These will include situations where actuation does not happen (e.g. due to power failure). Alternatively, this will include situations where actuation occurs, but reset of the coupling fails (e.g. due to power being in a 'continuous on' mode) and a manual reset, decoupling (e.g. by severing the coupling) or 'circuit break' is employed.

The invention will now be described further with reference to the accompanying drawings in which:
Figure 1a is a sectional side view of a first medicament dispenser described in WO-A-01/41849; Figure 1 b is a side view of the medicament dispenser of Figure 1 a; Figure 1c is sectional top view of part of the container seat of the medicament dispenser of Figures 1 a and 1b;
Figures 2a-2c are sectional views of a syringe dispenser described in WO-A-01/41849 in rest, fire and retract positions respectively;
Figure 3 is a sectional view of a decoupling means comprising a three-side cam;
Figure 4a is a sectional view of a decoupling means comprising a multi-lobed cam and 4b is a side perspective of part of the decoupling means;
Figure 5 is a sectional view of a decoupling means comprising a hinged beam;
Figures 6a to 6c show side perspective and sectional views of different decoupling means comprising a top and push crown;
Figure 7 shows a sectional view of decoupling means comprising a magneto rheological fluid;
Figures 8a and 8b are sectional views of wire-assisting means using a single lever;
Figure 9 is a sectional view of wire-assisting means comprising a stop lever;
Figure 10 is a sectional view of wire-assisting means comprising two wedge levers and showing a manual activation device;
Figure 11 is a sectional view of wire-assisting means comprising a scissored lever arrangement;
Figure 12 is a sectional view of wire-assisting means comprising a guide track and guide arm;
Figure 13 is a sectional view of wire-assisting means comprising a bent beam, in non-dispensing and dispensing positions;
Figure 14 is a side perspective of adjusting means of the invention comprising a drive shaft and drive plate;
Figure 15 is a side perspective and sectional view of adjusting means of the invention comprising a rack and wheel arrangement;
Figure 16 is a side perspective of adjusting means of the invention comprising a rising wedge design;
Figure 17a and 17b are sectional views of medicament dispenser having a storage and use configuration. Figure 17c is an alternative arrangement of a dispenser in storage configuration; and
Figure 18 is a sectional view of a disposable medicament container assembly comprising coupling wires.

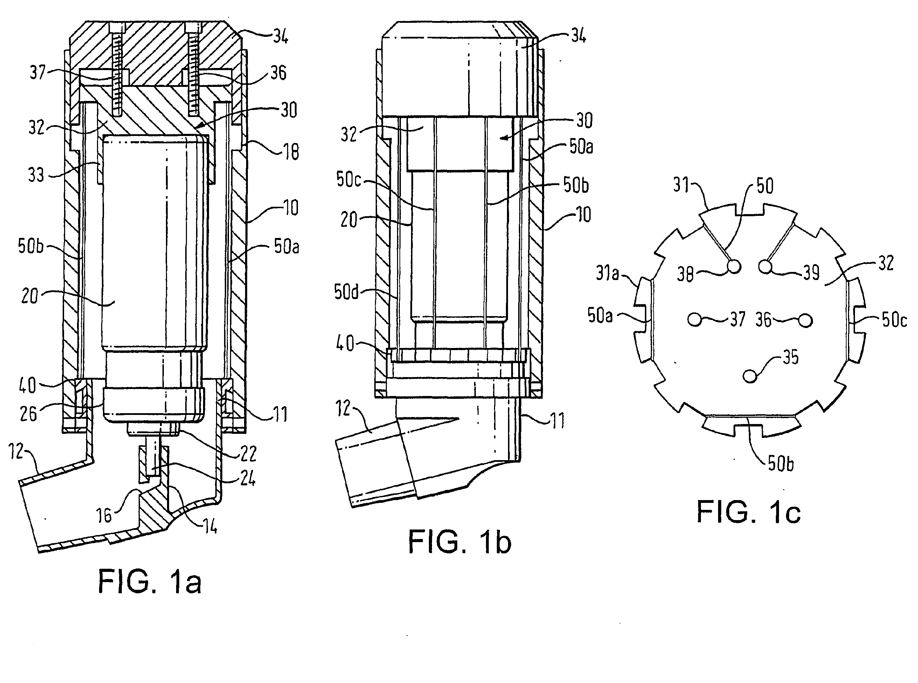

Figures 1a and 1b show a metered dose inhaler for the delivery of medicament for inhalation by a patient. The inhaler comprises a tubular housing 10 in which an aerosol container 20 is located. A dispensing outlet 12 leads laterally from the closed end of the housing 10. In the embodiment illustrated, the outlet 12 is in the form of a mouthpiece intended for insertion into the mouth of the patient but it may, if desired, be designed as a nozzle for insertion into the patient's nostril.

The aerosol container 20 has a valve dispensing mechanism 22 in the form of a slide valve. Valve stem 24 connects with a support 14. The support 14 is provided with an outlet passage 16 enabling dispensed dose to pass through to the dispensing outlet 12. It will be appreciated that dispensing of the dose requires the aerosol container 20 to be depressed to actuate the slide valve dispensing mechanism 22 and dispense medicament into the outlet 12 from which it can be inhaled by a patient.

It may be seen that the upper part of the aerosol container 20 is received by container seat 30. The container seat 30 is slidably movable within the housing along track 18 cut it into the wall of the housing 10. The container seat 30 comprises a first electrically conducting portion 32 having receiving side walls 33 to receive the aerosol container 20 and a second portion 34 which forms a manual actuation button for use as a manual override. The first portion 32 is fixed to the second portion 34 via screw connectors 36, 37. It may also be seen that the neck 26 of the valve 22 is received by electrically conducting ring 40 which fits around the neck 26 of the valve 22. The ring 40 is itself fixed to the lower part 11 of the housing 10. Plural lengths of shape memory alloy wire 50a, 50b, 50c, 50d connect the first portion 32 of the container seat 30 to the conducting ring 40. As will become apparent by inspection of Figure 1c, the plural lengths of wire 50a, 50b, 50c, 50d in fact, comprise a single wire 50 wrapped a number of times around the container seat 30 and ring 40. The wire 50 comprises a nickel-titanium alloy which contracts in response to the flow of electrical current therethrough. It may thus, be appreciated that when electrical current is passed through the plural lengths of wire 50a, 50b, 50c, 50d the container seat 30 and ring 40 will be drawn towards each other as the wire 50 contracts. Actuation of the valve dispensing mechanism 22 and dispensing of medicament dose will thereby result.

In the event of failure of electrical current flow it may be appreciated that the manual actuation button 34 may be manually pushed downwards to actuate the valve dispensing mechanism 22.

Figure 1 c shows a top view of the first portion 32 of the container seat 30. It may be seen that the first portion 32 is provided with crenellations 31, 31 a around its circumference. First and second points of wire attachment 38, 39 are provided such that a first end of shape memory alloy wire 50 is fixed to the first point of attachment 38. The wire 50 is then wrapped around first crenellation 31 and trailed down to the valve seat 40 with which it connects before leading up to second crenellation 31 around which it is wrapped and so on until multiple loops of wire 50a, 50b, 50c connect the ring 40 to the container seat 30. The end of the wire 50 is finally pulled taught and secured to end point of attachment 39. In alternative embodiments, a tensioning feature such as a screw tension mechanism may be included for use in tensioning the wire. Screw connector holes 36, 37 are provided to enable screw connection to the upper part 34 of the container seat 30. A power connector 35 is provided to enable connection of the first portion 32 and hence, wire 50 to an electrical power source.

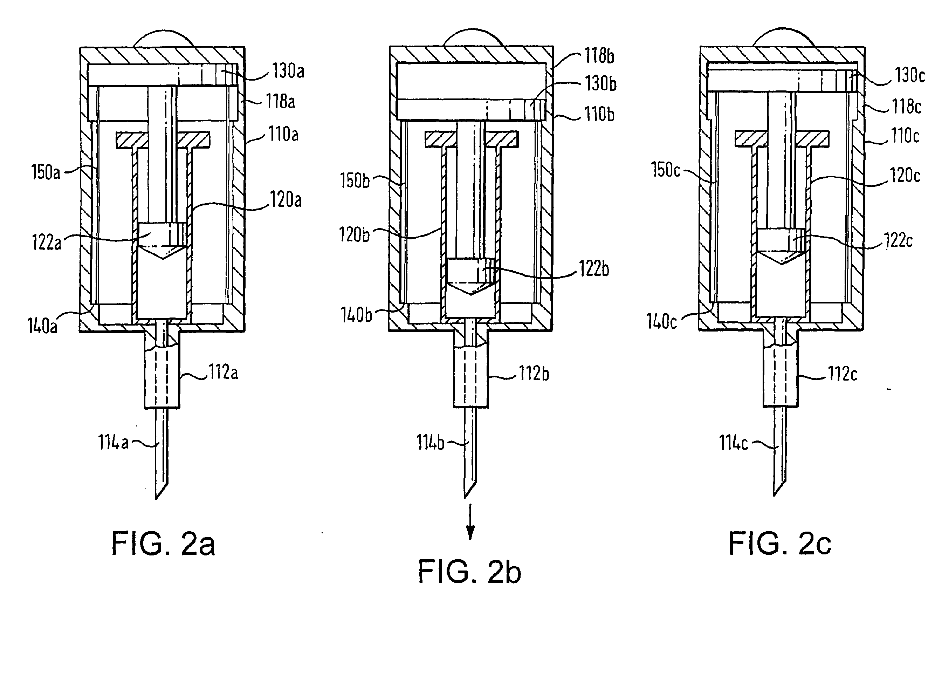

Figures 2a, 2b and 2c show schematic representations of a syringe for the delivery of medicament by injection in the rest, fine and retract positions respectively. The syringe comprises a hollow tubular barrel housing 110a-c defining a medicament chamber 120a-c. A dispensing outlet 112a-c leads laterally from the closed end of the chamber 120a-c. Connecting to outlet 112a-c there is provided hollow needle 114a-c through which medicament is dispensed.

The medicament chamber 120a-c has a drug mechanism 122a-c in the form of a plunger rod. The plunger rod 122a-c is movable within the chamber 120a-c to vary the volume thereof and hence, to expel any medicament preparation contained therein (generally in solution form) through the outlet 112a-c to the hollow needle 114a-c. It will thus, be appreciated that dispensing of the medicament requires the plunger 122a-c to be depressed to dispense medicament into the outlet 112a-c and thence to the hollow needle 114a-c through which it is injectable through the skin of a patient.

It may be seen that the upper part 124a-c of the plunger 122a-c is received by actuator seat 130a-c. The actuator seat 130a-c is slidably movable within the housing 110a-c along track 118a-c cut it into the wall of the housing 110a-c. Shape memory alloy wire 150a-c connects actuator seat 130a-c to a fixed anchor position 140a-c at the base of the housing 110a-c. The wire 150a-c comprises a nickel titanium alloy which contracts in response to the flow of electrical current therethrough. It may thus, be appreciated that when electrical current is passed through the wire 150a-c the actuator seat 130a-c and hence the plunger 122a-c will be drawn down towards the fixer anchor position 140a-c as the wire 150a-c contracts. Actuation of the plunger dispensing mechanism 122a-c and dispensing of the medicament through the outlet 112a-c and hollow needle 114a-c will thereby result.

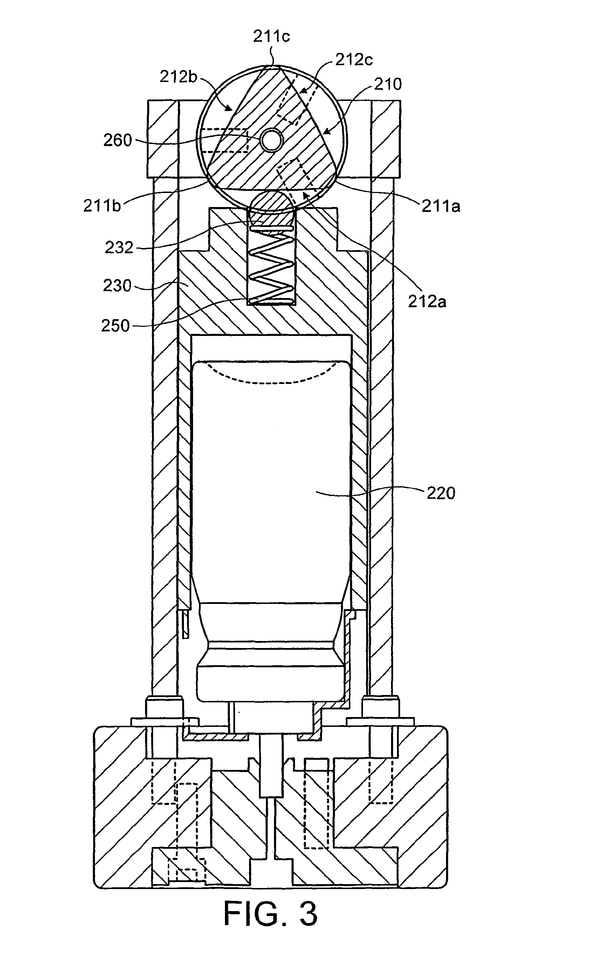

In Figure 3 the decoupling means comprises a three sided cam 210 having three peaks 211a-c and three resting sides 212a-c. The cam cooperates with a medicament container 220 via a carriage 230. Located between the cam 210 and the carriage 220 is a ball bearing 232 and a spring 250. A number of Shape Memory Alloy wires (not shown) are radially attached to the cam 210 and to a fixed point on the dispenser (not shown). As actuation begins and the wires (not shown) connected to the cam 210 contract, peak 211 a begins to exert a downward force on the ball 232 which in turn exerts a downward force on carriage 230.The medicament container 220 is moved downwards into a dispensing position. As peak 211 a is passed, the cam 210 quickly rotates further, spring 250 exerting an upward force on ball 232 causing the cam 210 to settle in a resting position on resting side 212c. The medicament container 220 returns to its non-dispensing and refilling position. Coupling wires (not shown) will then continue to relax until their rest position is reached. A clutch 260 at the centre of cam 210 rotates in a reverse direction to rewind coupling wires (not shown) around cam 210 until taut and ready to drive the cam for a further actuation.

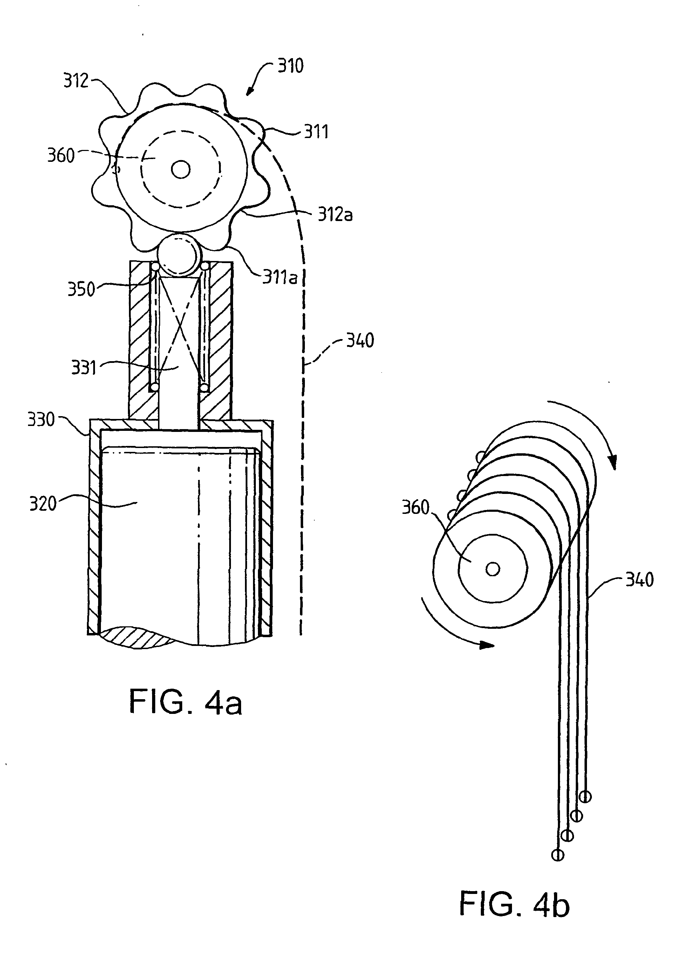

In Figure 4a the decoupling means comprises a multi-lobed cam 310, having a number of lobes 311 and depressions 312. The cam cooperates with a medicament container 320 via a housing 330 and stem 331. Located between the stem 331 and the cam 310 is a ball bearing 332 which rests in one of the depressions 312. A number of Shape Memory Alloy wires 340 are radially attached to the cam 310 and to a fixed point on the dispenser (not shown). As actuation begins and the wires 340 connected to the cam 310 contract, lobe 311 a begins to exert a downward force on the ball 332 which in turn exerts a downward force on stem 331.The medicament container 320 is moved downwards into a dispensing position. As the peak of lobe 311a is passed, the cam 310 quickly rotates further, spring 350 exerting an upward force on ball 332 causing the ball 332 to settle in a resting position in depression 312a. The medicament container 320 returns to its non-dispensing and refilling position. Coupling wires 340 will then continue to relax until their rest position is reached. A clutch 360 at the centre of cam 310 rotates in a reverse direction to rewind coupling wires 340 around cam 310 until taut and ready to drive the cam for a further actuation (Figure 4B).

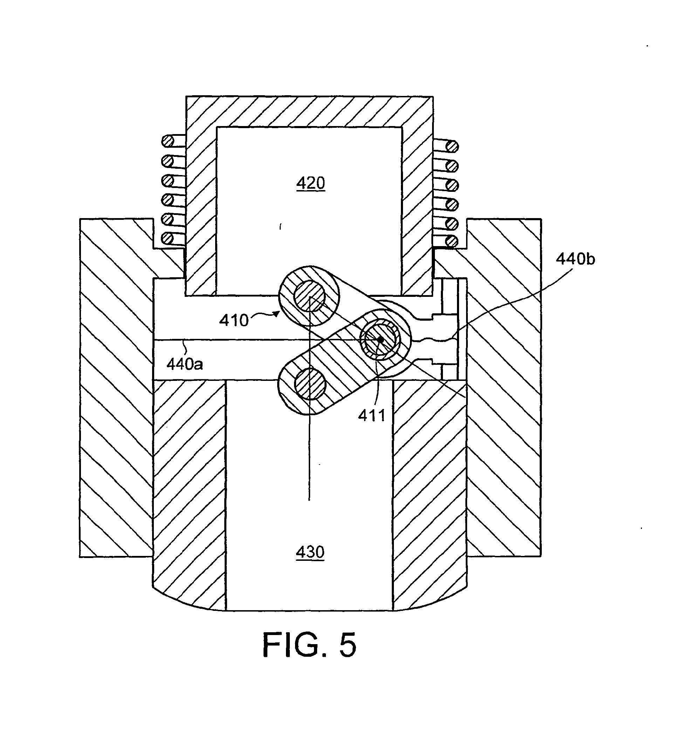

In Figure 5, the decoupling means for use in a medicament dispenser as hereinbefore described comprises a scissor beam 410 hinged at its centre point 411, the upper end of the beam 410 attached to a fixed point in the housing 420 and the lower end of the beam 410 attached to a medicament container 430. Two wires 440a, 440b are attached to the beam 410 at the hinged point 411, the other ends of the wires attached to opposite sides of the housing. In the rest position, one wire will be extended so that it is taut, while the other wire is slack. As the taut wire begins to contract the beam begins to straighten and exerts a downwards force on the dispensing can moving it downwards. At the point when the beam is straight, the dispensing can will be in the dispensing position and the active component will be released. As soon as the straight position of the beam is passed, the beam is forced into its opposite rest position by a recovering valve spring (not shown) that becomes free of the force acting upon it, allowing the dispensing can to refill. The wires will intiependently relax leaving one taut wire and one slack wire.

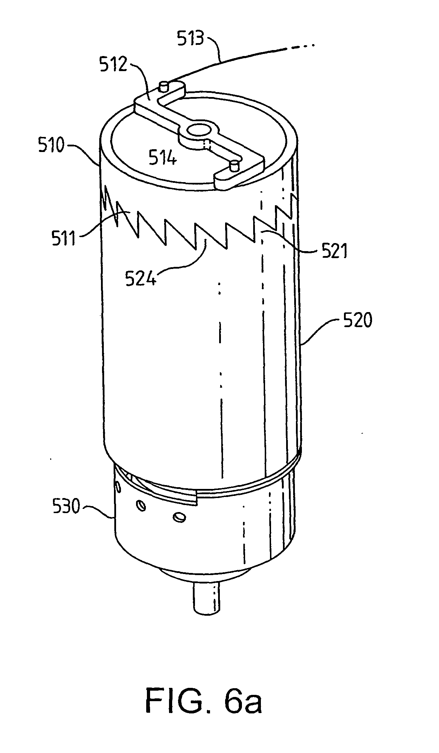

In Figure 6a, decoupling means suitable for use in a medicament dispenser as hereinbefore described comprises top crown 510 provided with a plurality of teeth 511. Attached to the upper surface of top crown 510 is an over-running dutch 512 attached to which are one or more coupling wires 513. Located beneath the top crown 510 is a push crown 520 provided with a plurality of teeth 521 which engage with the teeth 511 of the top crown 510. The push crown 520 houses a medicament dispenser 530. As the coupling wires 513 contract during activation of the device, the top crown 510 rotates in a clockwise direction. As teeth 511 move over teeth 521, a downward force is exerted on the push crown 520 which moves medicament dispenser 530 down into a dispensing position. Once tooth peak 514 and tooth peak 524 are aligned, the medicament dispenser 530 has reached the dispensing position and the active component is dispensed. Slight further rotation of top crown 510 causes the tooth peaks 514, 524 to disengage and the medicament dispenser 530 is returned to its rest position with the aid of a compressed valve spring (not shown) which becomes free of the force acting on it. As coupling wires 513 relax, the clutch 512 rotates in an anticlockwise direction returning to its start position in readiness for the next actuation.

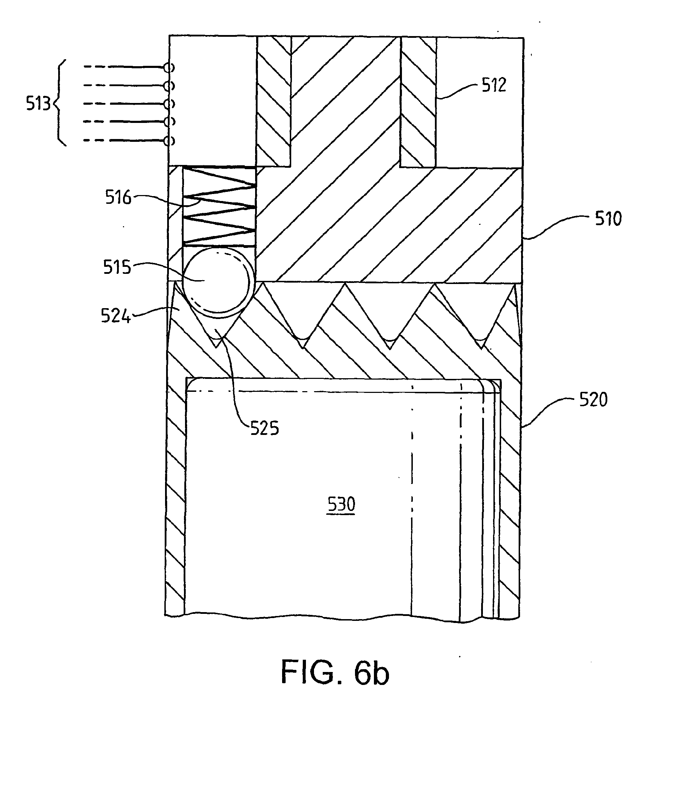

Figure 6b shows an alternative device to that shown in Figure 6a. The decoupling means comprises a push crown 520 housing a medicament container 530, the push crown 520 being provided with a plurality of teeth 521. The top crown 510 comprises a clutch 512 and a plurality of coupling wires 513. Instead of being provided with teeth, the top crown is provided with a ball 515 and spring 516 arrangement, the ball 515 engaging with trough 525. As the device is activated, coupling wires 513 contract causing top crown 510 to rotate. As top crown 510 rotates, a downward force is exerted by ball 515 on tooth peak 524. The push crown 520 is moved downwards moving the medicament dispenser 530 into a dispensing position. As ball 515 is vertically aligned over peak 524 the medicament dispenser 530 reaches the dispensing position and the active component is dispensed. Slight further rotation of top crown 510 cause the ball 515 and peak 524 to disengage, the medicament dispenser 530 returns to its rest position and ball 515 comes to rest in the trough adjacent to trough 525 (not shown).

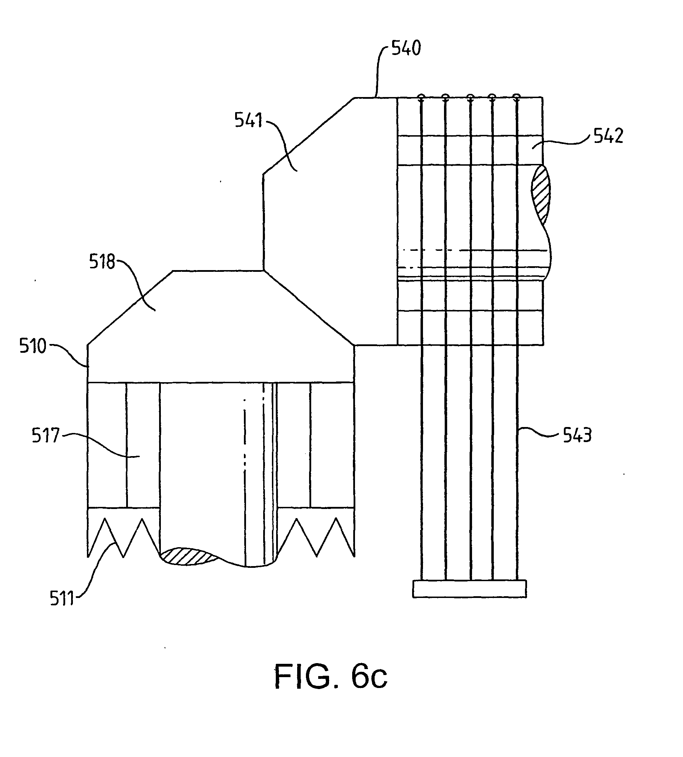

A further arrangement is shown in Figure 6c which uses a bevel gear arrangement. This allows the wires to be mounted vertically in the device, the vertical motion being transferred to radial motion by means of the bevel gear. Top crown 510 comprises clutch 517 and a set of teeth 511. The top crown 510 engages with a push crown (not shown) as described in Figure 6a. The upper part 518 of top crown 510 is of a shape adapted to engage with a corresponding shape 541 on bevel gear 540. Bevel gear 540 comprises a dutch 542 and a number of coupling wires 543. When the device is activated, coupling wires 543 contract and cause bevel gear 540 to rotate. Shape 541 engages with shape 518 causing top crown 510 to rotate. The decoupling device then continues to work as described in Figure 6a.

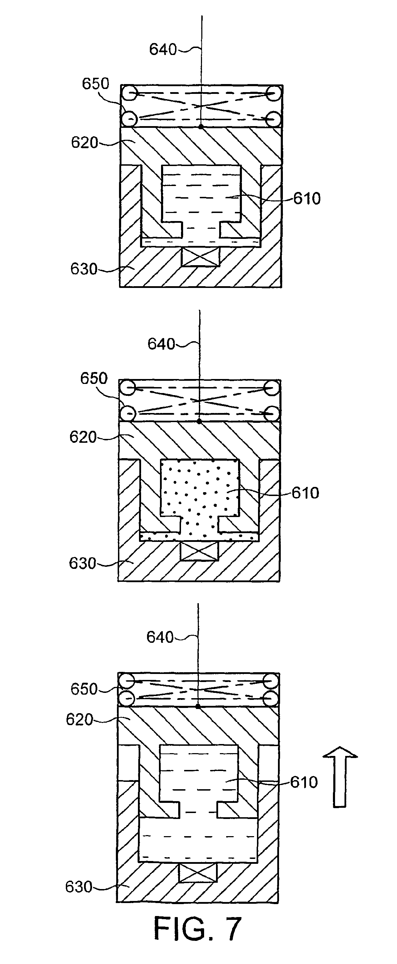

Figure 7 shows decoupling means comprising a magneto rheological fluid 610 contained within a space created between a plunger ring 620 and a cup ring 630. One or more coupling wires 640 are fixed between the plunger ring and a medicament container (not shown). When a current is placed near the magneto rheological fluid 610, the fluid 610 becomes solid. As the wires 640 contract, the medicament container (not shown) is pulled down into a dispensing position. When the current is removed, the solid 610 becomes fluid again, the plunger 620 moves quickly up under the force of a valve spring (not shown) allowing the medicament container (not shown) to return to its rest position. As the wires cool, the compression spring 650 moves the plunger back to its resting position.

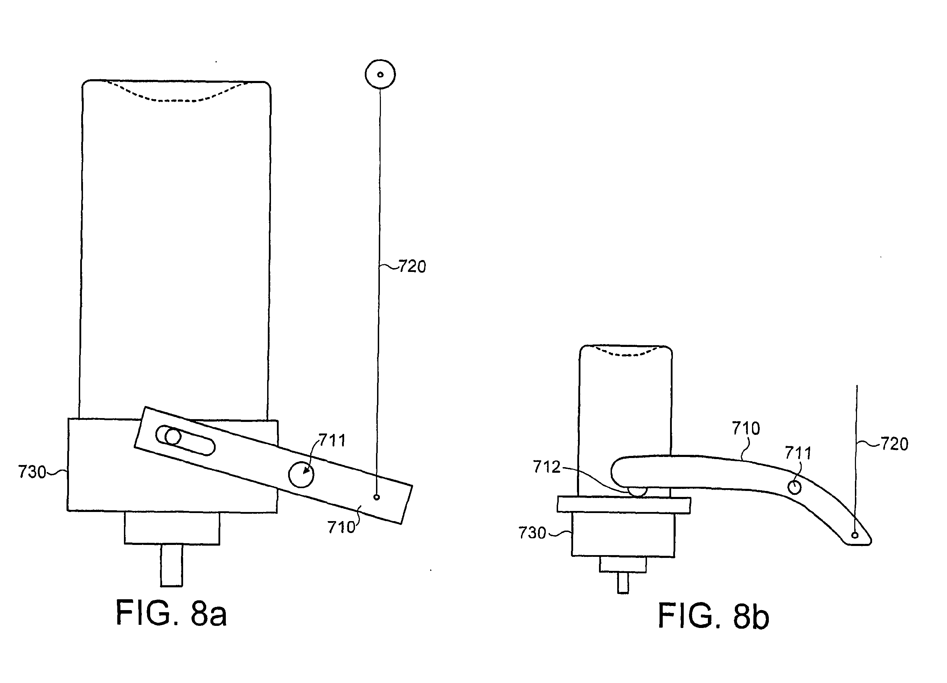

Figure 8a shows wire-assisting means comprising a single lever 710 attached at one end to a coupling wire 720, the other end of the wire attached to the housing (not shown). The other end of the lever 710 is attached to the medicament container 730, the lever 710 and the container 730 being movable with respect to each other. A pivot point 711 exist at a point between the attached wire 720 and the centre of the lever 710. As the wire 720 contracts, the lever 710 rotates around the pivot point 711, the other end of the lever moving down and rotating with respect to the container 730 which is moved into a dispensing position.

An alternative arrangement using a single lever is shown in Figure 8b. In this arrangement, the half of the lever 710 attached to wire 720 is of a curved nature and comprises the pivot point 711. The other end of the lever 710 has a protrusion 712 which engages with the medicament container 730. As the wire 720 contracts, the lever 710 pivots around the pivot point 711, the protrusion 712 engaging with the container 730 and moving it into a dispensing position.

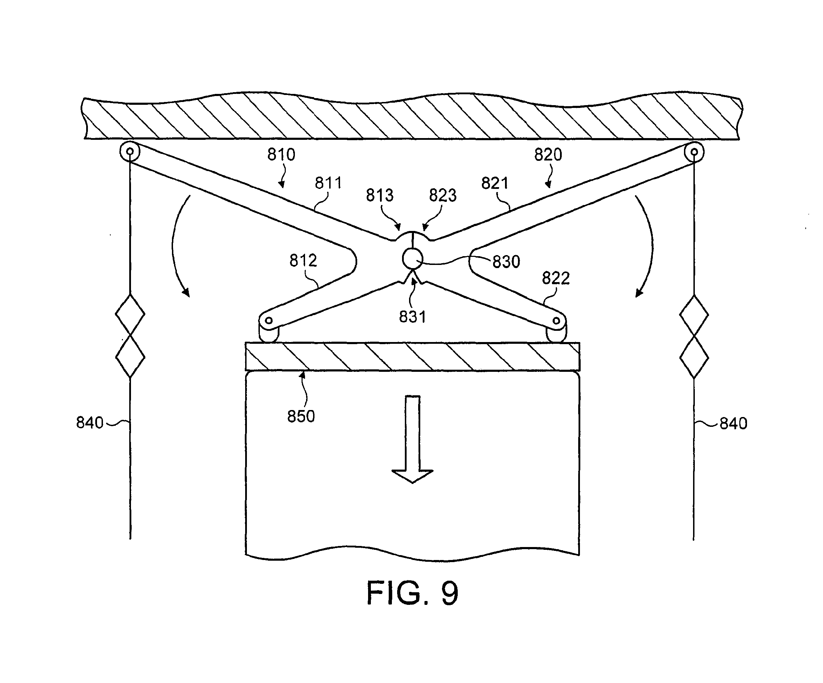

Figure 9 shows wire-assisting means comprising a first 810 and second 820 lever, each of which comprises a first 811, 821 and second 812, 822 lever arm, the second lever arm 812, 822 being shorter than the first lever arm 811, 821. The first and second arms of each lever are joined at a first end of each 813, 823 to form a pivot point 830. The second end of the first lever arm 811, 821 is attached to a coupling wire 840, the other end of the coupling wire being attached to the main body of the device or an anchor point with in it (not shown). The second lever arm 812, 822 of each lever rests on a feature 840 attached to the can. A notch 831 is provided at the pivot point 830 to enable the first and second lever 810, 820 to pivot around said pivot point 830. As the wires 840 contract, the first lever arms 811, 821 of the first and second levers 810, 820 are pulled downwards forcing the first and second levers 810, 820 to rotate around the pivot point 830 until the notch 831 closes and a stop position is reached. As the two levers 810 and 820 rotate, the second lever arm 812, 822 of each lever pushes downward on top plate 850, moving the medicament container into a dispensing position.

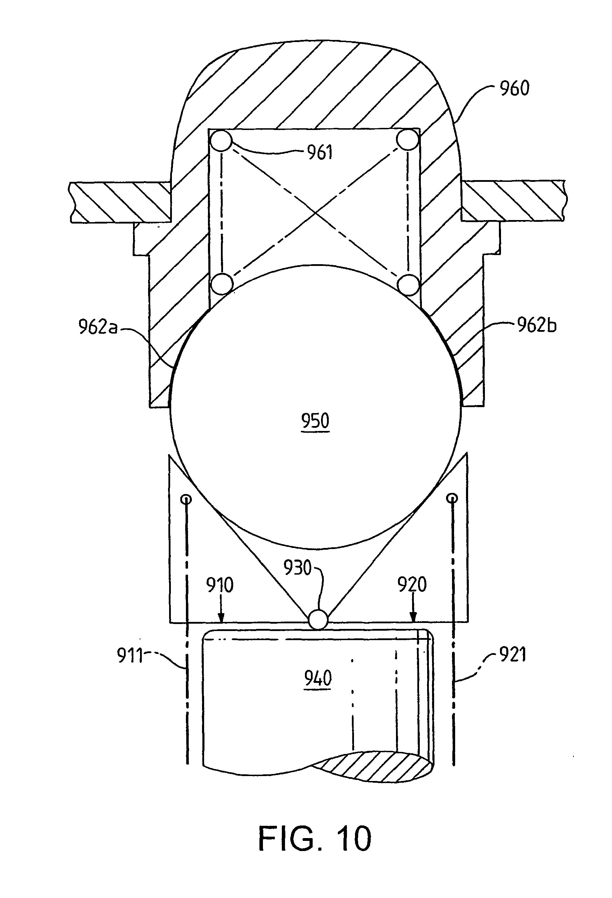

Figure 10 shows wire-assisting means comprising a first 910 and second 920 lever joined at a pivot point 930 and resting on a dispensing can 940. Coupling wires 911, 921 are attached to the levers 910, 920, the other end of coupling wires 911, 921 being attached to a fixed point on the dispenser (not shown). As the wires 911, 921 contract, the two levers 910, 920 pivot around the pivot point 930 and push down on the dispensing can 940. The can is moved downwards into a dispensing position. A further aspect of this arrangement provides a mechanism for dispensing the can should the contraction of the wires fail. A ball 950 is provided which rests within the two levers 910, 920. A button 960 is provided above the ball 950 and the button 960 housing a spring 961 to hold the button 960 in a non-dispensing mode. Upon pushing on the button 960, the spring 961 is compressed and causes the lower edge the button 962a, 926b to act upon the ball 950. The ball 950 in turn moves down into the wedge-shaped levers 910, 920, causing the two levers 910, 920 to pivot around the pivot point 930 and push down on the dispensing can 940.

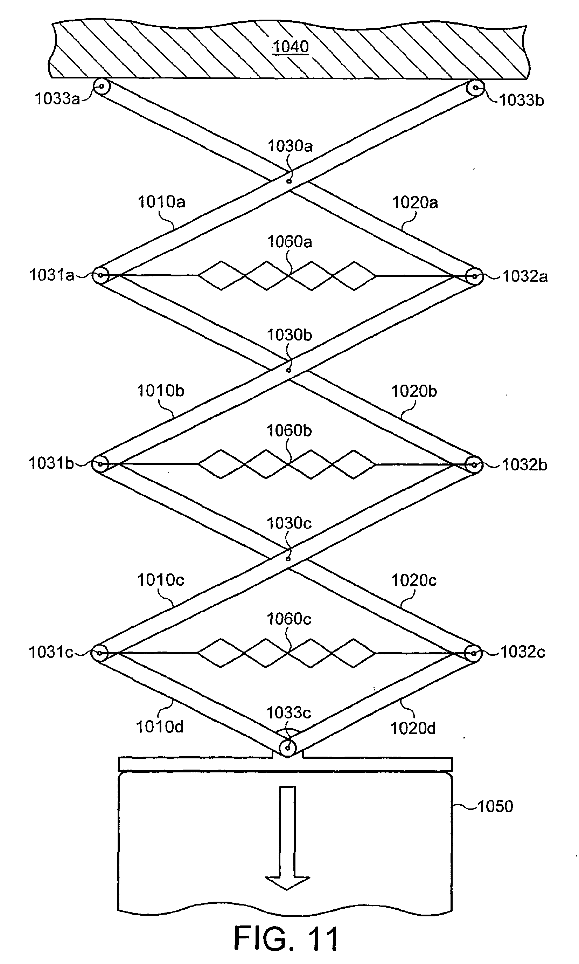

Figure 11 shows wire-assisting means comprising a number of first levers 1010a-c and a number of second levers 1020a-c arranged in a criss-cross manner. At the point where levers 1010a, 1020a, 1010b, 1020b and 1010c, 1020c cross, pivot points 1030a-c are formed. Pivot points 1031 a-b and 1032a-b are also formed where the end of the lever join. The upper ends of levers 1010a and 1020a are pivotally joined 1033a, 1033b to a fixed point in the dispenser 1040. The lower ends of levers 1010c and 1020c are joined to two further levers 1010d and 1020d, forming pivot points 1030c and 1032c. The other two ends of levers 1010d and 1020d are joined and pivotally attached 1033c to a fixed point on the medicament container 1050. Coupling wires 1060a-c extend between points 1031a and 1032a, 1031b and 1032b and 1031c and 1032c. Upon activation of the device, the coupling wires 1060a-c contract causing the whole lever system to elongate and causing a downward movement of the dispensing can 1050 into a dispensing position.

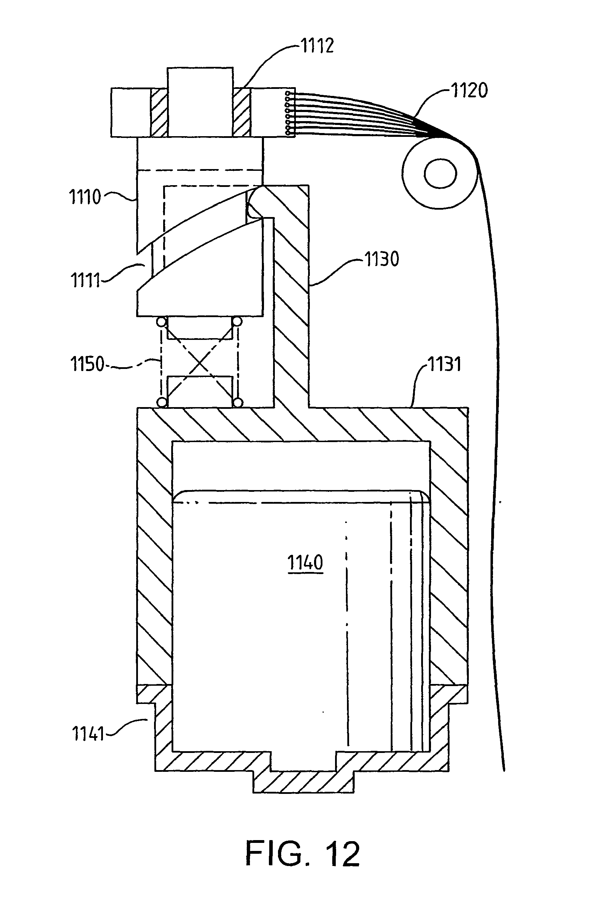

Figure 12 shows wire-assisting means comprising a rotatable cylinder 1110 comprising a downwardly spiralling guide track 1111 and a clutch 1112 at the centre thereof. A number of coupling wires 1120 are radially attached to the top of the cylinder 1110, the other end of the coupling wires 1120 being attached to a fixed point of the dispenser (not shown). A guide arm 1130 is connected to a cap 1131, the cap 1131 fitting over a medicament container 1140 which sits in a collar 1141. Located between the bottom of the cylinder 1110 and the top of the cap 1131 is a spring 1150. The guide arm 1130 is locatable within the guide track 1111. As the device is activated, the coupling wires 1120 contract, causing cylinder 1110 to rotate. As it rotates, the spiralling guide track 1111 causes the guide arm 1130 to move downwards in a vertical manner, the cap 1131 causing the medicament container 1140 to move to a dispensing position.

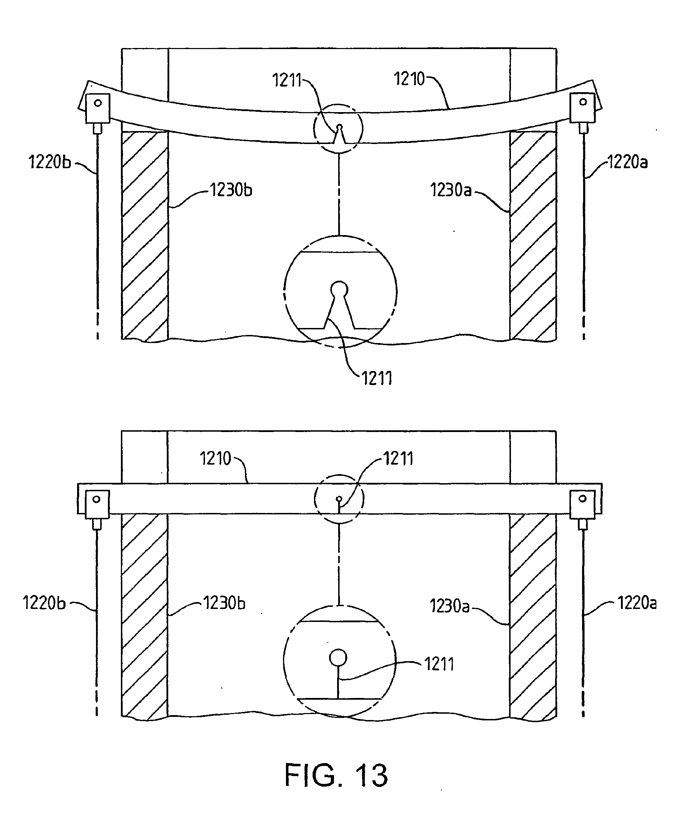

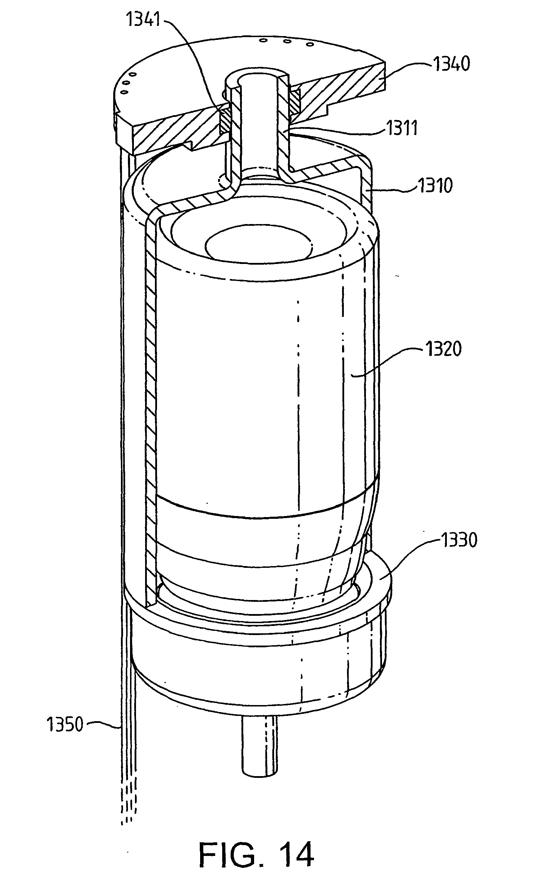

Figure 13 shows wire-assisting means comprising a bowed beam 1210, the beam 1210 having a notch 1211 cut in the lower surface of the beam in the centre. A coupling wire 1220a,b is attached to either end of the beam, the other end of each coupling wire 1220a,b being attached to a fixed point in the dispenser (not shown). The beam 1210 rests at either end on two vertical carriages 1230a,b which in turn are connected to the medicament container (not shown). As the device is activated, coupling wires 1220a,b contract, both ends of the beam 1210 are pulled downwards until the beam is straight and the notch 1211 closed. A downward pressure is exerted on the two carriages 1230a,b which in turn forces medicament container (not shown) into a dispensing position. As the wires 1220a, b again relax, the beam 1210 rebends and the medicament container (not shown) returns to the non-dispensing position. Figures 14 to 16 show different embodiments of the present invention.

Figure 14 shows adjusting means comprising a carriage 1310 having a drive shaft 1311 extending from the top thereof. Carriage 1310 houses a medicament container 1320 which sits on a container seat 1330. Carriage 1310 also rests on the container seat 1330. The drive shaft 1311 of the carriage 1310 is locatable within an opening in a drive plate 1340, which has a clutch 1341 at the centre thereof. A number of coupling wires 1350 extend from the drive plate to a fixed point in the dispenser (not shown). The clutch 1341 permits the shaft 1311 to find its rest position without applying any load. Once the device is activated, the clutch 1341 grips the shaft 1311 and the wires 1350. The drive plate 1340, carriage 1310 and medicament container 1320 are pulled down into a dispensing position. Once the wires 1350 have fully contracted and the medicament container 1320 fired, the clutch 1341 releases the drive shaft 1311 and allows the medicament container 1320 to reset under a valve spring (not shown). In a variation on the device shown in Figure 14, the wires 1350 may be fixed at an a slight angle such that contraction of the wires causes the dutch 1341 and drive shaft 1311 to engage and as the wires 1350 started to cool, the clutch 1341 would be released.

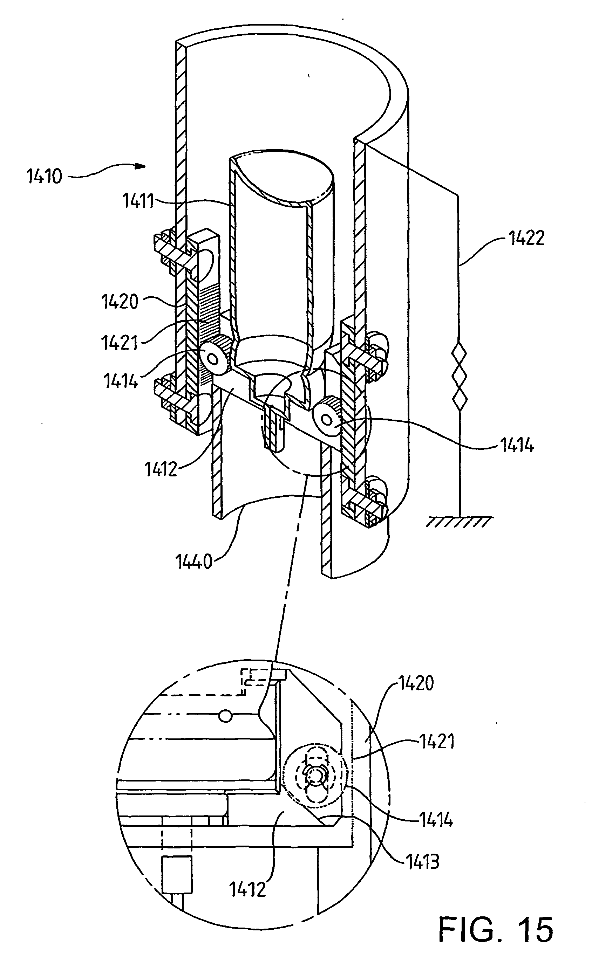

Figure 15 shows adjusting means comprising a rack and wheel mechanism. A can assembly 1410 comprises a dispensing can 1411, a collar 1412 having a rack profile 1413, and a plurality of wheels 1414, preferably four. The can assembly 1410 is located in a body 1420 which contains two vertical racks 1421 on opposing inside surfaces of the body and one or more coupling wires 1422 attached to the outside of the body 1420 at a position defined by the wire length. The position of the can assembly 1410 in the body 1420 is defined by a stem block (not shown) in the mouth piece 1440. The racks 1421 and wheels 1414 find a position where they mesh together. Upon activation of the device, the wire 1422 begins to contract causing a vertical displacement of the body 1420. As this displacement occurs, the wheels 1414 mesh with the rack 1421 on the body 1420 and the rack profile 1413 on the collar 1412. The wheels 1414 lock enabling the can assembly 1410 to move with the body 1420 causing the can 1411 to fire. After firing, activation is stopped, the wires 1422 begin to relax and the body 1420 and can assembly 1410 return to their resting position.

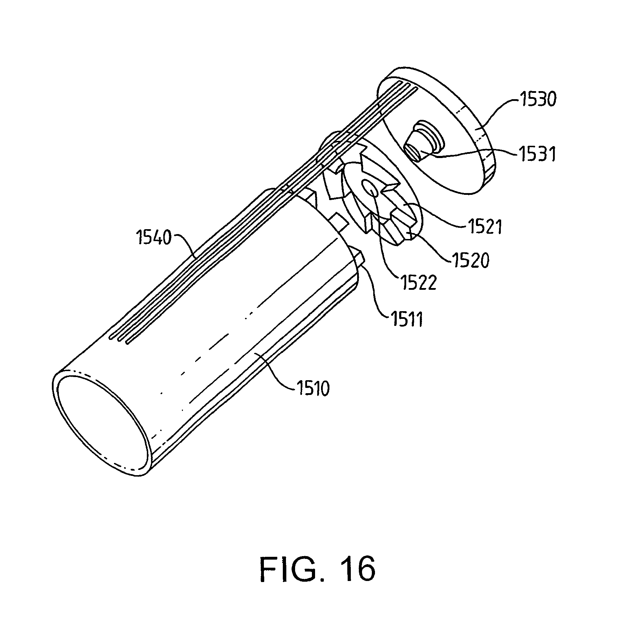

Figure 16 shows adjusting means comprising a carriage 1510 closed at one end and having located on that end a number of truncated wedge-shaped teeth 1511. The carriage is adapted to house a medicament container (not shown), having a number of interference wedges 1512 to hold the container in place. The wedge-shaped teeth 1511 cooperate with a drive wedge 1520 having a plurality of teeth 1521, equal to the number of wedge shaped teeth 1511. A hole 1522 at the centre of the drive wedge 1520 has a shaft 1531 of a drive plate 1530 inserted therein. A number of coupling wires 1540 extend from the drive plate 1530 to a fixed point on the dispenser (not shown). As the carriage 1510 is rotated from a load position to the engage position, the teeth 1511 on the carriage engage with the wedge shaped teeth 1521 on the drive wedge 1520. As the carriage 1510 is rotated further, the medicament container (not shown) is moved into the dispensing position and will fire. Once fired the carriage 1510 is rotated back to the rest position.

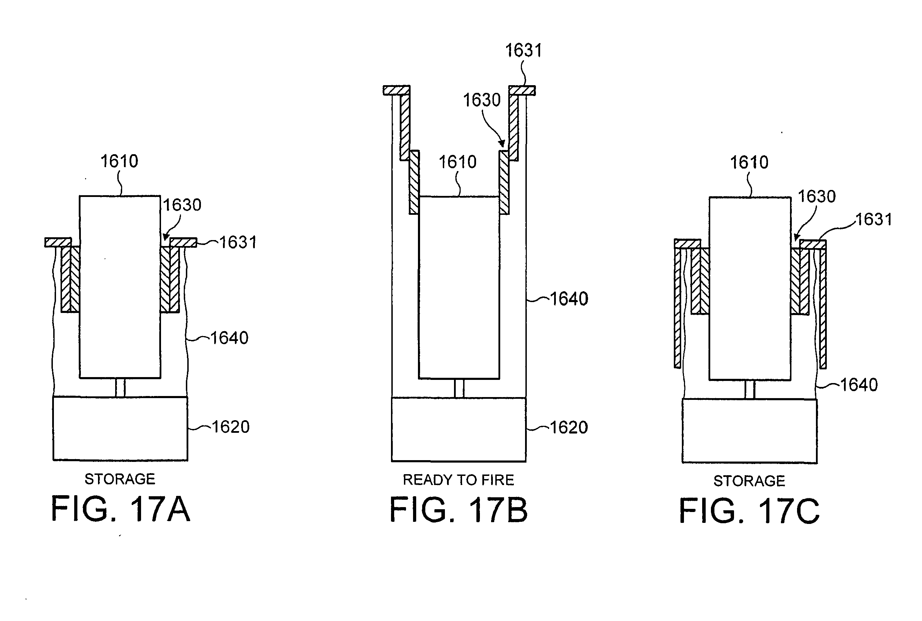

Figures 17a and 17b show a medicament device having a storage (Figure 17a) and a use (Figure 17b) configuration. The medicament container 1610 sits in a container seat 1620 and has surrounding it a plurality of concentric cylinders 1630. Attached between the outer cylinder 1631 and the container seat 1620 are at least two coupling wires 1640; in the storage configuration, the coupling wires 1640 are slack. When the device is to be used, the concentric cylinders 1630 are extended such that the coupling wires 1640 are now taut. As the coupling wires 1640 are activated and contract, the medicament container 1610 dispenses its load. Figure 17c shows a similar device in the storage configuration wherein the outer cylinder 1631 is of a double walled construction.

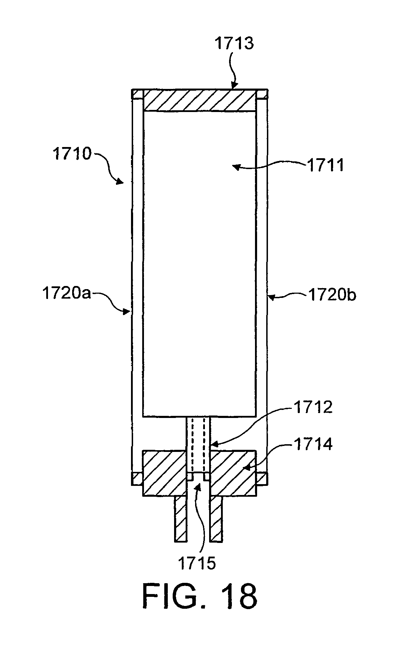

Figure 18 shows a disposable medicament container assembly 1710 comprising the medicament container 1711, having a stem 1712. Located at the top of the medicament container 1711 is a top cap 1713. At the bottom of the medicament container 1711 is a stem drive 1714 having an opening 1715 therein to accommodate the stem 1712. A coupling mechanism 1720a,b is provided between the top cap 1713 and the stem drive 1714. When the device is activated, the coupling mechanism 1720a,b contracts forcing the stem 1712 through the stem drive 1714 and dispensing the dose. Once the medicament container 1711 is empty, the entire assembly is discarded and replaced with a new assembly.

In alternative embodiments, the powder metering and transport system and the aerosolisation system may be actuable through a coupled SMA wire assembly. For example, the SMA wire assembly may sequentially actuate metering, transport and aerosolisation.

It may be appreciated that any of the parts of the dispenser or actuator which contact the medicament suspension may be coated with materials such as fluoropolymer materials (e.g. PTFE or FEP) which reduce the tendency of medicament to adhere thereto. Any movable parts may also have coatings applied thereto which enhance their desired movement characteristics. Frictional coatings may therefore be applied to enhance frictional contact and lubricants (e.g. silicone oil) used to reduce frictional contact as necessary.

The medicament dispenser of the invention is suitable for dispensing medicament, particularly for the treatment of respiratory disorders such as asthma and chronic obstructive pulmonary disease (COPD).

Appropriate medicaments may thus be selected from, for example, analgesics, e.g., codeine, dihydromorphine, ergotamine, fentanyl or morphine; anginal preparations, e.g., diltiazem; antiallergics, e.g., cromoglycate (eg as the sodium salt), ketotifen or nedocromil (eg as the sodium salt); antiinfectives e.g., cephalosporins, penicillins, streptomycin, sulphonamides, tetracyclines and pentamidine; antihistamines, e.g., methapyrilene; anti- inflammatories, e.g., beclomethasone (eg as the dipropionate ester), fluticasone (eg as the propionate ester), flunisolide, budesonide, rofleponide, mometasone (eg as the furoate ester), ciclesonide, triamcinolone (eg as the acetonide), 6α, 9α-difluoro-11β-hydroxy-16α-methyl-3-oxo-17α-propionyloxy-androsta-1,4-diene-17β-carbothioic acid S-(2-oxo-tetrahydro-furan-3-yl) ester or 6α, 9α-Difluoro-17α-[(2-furanylcarbonyl)oxy]-11β-hydroxy-16α-methyl-3-oxo-androsta-1,4-diene-17β-carbothioic add S-fluoromethyl ester; antitussives, e.g., noscapine; bronchodilators, e.g., albuterol (eg as free base or sulphate), salmeterol (eg as xinafoate), ephedrine, adrenaline, fenoterol (eg as hydrobromide), formoterol (eg as fumarate), isoprenaline, metaproterenol, phenylephrine, phenylpropanolamine, pirbuterol (eg as acetate), reproterol (eg as hydrochloride), rimiterol, terbutaline (eg as sulphate), isoetharine, tulobuterol or 4-hydroxy-7-[2-[[2-[[3-(2-phenylethoxy)propyl]sulfonyl]ethyl]amino]ethyl-2(3H)-benzothiazolone; PDE4 inhibitors eg cilomilast or roflumilast; leukotriene antagonists eg montelukast, pranlukast and zafirlukast adenosine 2a agonists, e.g. 2R,3R,4S,5R)-2-[6-Amino-2-(1S-hydroxymethyl-2-phenyl-ethylamino)-purin-9-yl]-5-(2-ethyl-2H-tetrazol-5-yl)-tetrahydro-furan-3,4-diol (e.g. as maleate); α₄ integrin inhibitors e.g. (2S)-3-[4-({[4-(aminocarbonyl)-1-piperidinyl]carbonyl}oxy)phenyl]-2-[((2S)-4-methyl-2-{[2-2-methylphenoxy) acetyl]amino}pentanoyl)amino] propanoic acid (e.g. as free acid or potassium salt), diuretics, e.g., amiloride; anticholinergics, e.g., ipratropium (eg as bromide), tiotropium, atropine or oxitropium; homones, e.g., cortisone, hydrocortisone or prednisolone; xanthines, e.g., aminophylline, choline theophyllinate, lysine theophyllinate or theophylline; therapeutic proteins and peptides, e.g., insulin or glucagons. It will be clear to a person skilled in the art that, where appropriate, the medicaments may be used in the form of salts, (e.g., as alkali metal or amine salts or as acid addition salts) or as esters (e.g., lower alkyl esters) or as solvates (e.g., hydrates) to optimise the activity and/or stability of the medicament

Preferred medicaments are selected from albuterol, salmeterol, fluticasone propionate and bedomethasone dipmpionate and salts or solvates thereof, e.g., the sulphate of albuterol and the xinafoate of salmeterol.

Medicaments can also be delivered in combinations. Preferred formulations containing combinations of active ingredients contain salbutamol (e.g., as the free base or the sulphate salt) or salmeterol (e.g., as the xinafoate salt) or formoterol (eg as the fumarate salt) in combination with an antiinflammatory steroid such as a beclomethasone ester (e.g., the dipropionate) or a fluticasone ester (e.g., the propionate) or budesonide. A particularly preferred combination is a combination of fluticasone propionate and salmeterol, or a salt thereof (particularly the xinafoate salt). A further combination of particular interest is budesonide and formoterol (e.g. as the fumarate salt).

It will be understood that the present disclosure is for the purpose of illustration only and the invention extends to modifications, vanations and improvements thereto within the scope of the appended claims.

## Claims

1. A medicament dispenser comprising a housing; a medicament container (1320;1411) having a dispensing mechanism; a container seat (1330) for receipt of the container; an anchor station on the housing or connecting therewith; and drive means (1350;1422;1540) capable of moving the container seat relative to the anchor station to actuate the dispensing mechanism, the drive means comprising a coupling (1350;1422;1540), which deforms, or undergoes a phase transition in response to the application of that energy, electrical current energy, electrical field energy or magnetic field energy wherein the medicament dispenser further comprises adjusting means (1310,1311,1340,1341;1414,1421;1510,1520,1530) adapted to enable medicament containers of varying sizes to be received by the housing.

2. A medicament dispenser according to claim 1, wherein the adjusting means comprises variable engaging means.

3. A medicament dispenser according to claim 2, wherein the variable engaging means comprises a first engager located on a first part of the medicament dispenser and a second engager located on a second part of the medicament dispenser, wherein the first and second engager are engageable with the medicament container.

4. A medicament dispenser according to claim 2, wherein the adjusting means comprises a clutch and drive plate mechanism (1340,1141), which interconnects with a carriage shaft (1311) which houses the medicament container (1320).

5. A medicament dispenser according to claim 2, wherein the adjusting means comprises a rack and wheel mechanism (1414,1421).

6. A medicament dispenser according to claim 2, wherein the adjusting means comprises a flexible bag containing a viscous fluid, suitably located in the base of the housing.

7. A medicament dispenser according to claim 2, wherein the adjusting means comprises a drive wedge mechanism (1510,1520,1530).

8. A medicament dispenser according to any of claims 1 to 7, wherein the dispensing mechanism is selected from the group consisting of a valve, pump or plunger mechanism.

9. A medicament dispenser according to any one of the preceding claims 5, wherein the coupling comprises a shape memory alloy.

10. A medicament dispenser according to claim 9, wherein the shape memory alloy is a nickel-titanium alloy.

11. A medicament dispenser according to any of claims 1 to 10, wherein the medicament container is an aerosol container.

## Patentansprüche

1. Medikamentenspender mit einem Gehäuse; einem Medikamentenbehälter (1320; 1411), der einen Abgabemechanismus aufweist; einem Behältersitz (1330) zur Aufnahme des Behälters; einer Verankerungsstation an dem Gehäuse oder damit verbunden; und einer Antriebseinrichtung (1350; 1422; 1540), die in der Lage ist den Behältersitz relativ zu der Verankerungsstation zu bewegen, um den Abgabemechanismus zu betätigen, wobei die Antriebseinrichtung eine Kopplung (1350; 1422; 1540) umfasst, die sich verformt, oder eine Phasenumwandlung erfährt, als Antwort auf die Anwendung von Wärmeenergie, elektrischer Stromenergie, elektrischer Feldenergie oder magnetischer Feldenergie, wobei der Medikamentenspender ferner eine Anpassungseinrichtung (1310; 1311; 1340; 1341; 1414; 1421; 1510; 1520; 1530) umfasst, die geeignet ist zuzulassen, dass Medikamentenbehälter unterschiedlicher Größen durch das Gehäuse aufgenommen werden.

2. Medikamentenspender nach Anspruch 1, bei dem die Anpassungseinrichtung eine variable Eingriffseinrichtung umfasst.

3. Medikamentenspender nach Anspruch 2, bei dem die variable Eingriffseinrichtung einen ersten Eingreifer, der sich an einem ersten Teil des Medikamentenspenders befindet, und einen zweiten Eingreifer umfasst, der sich an einem zweiten Teil des Medikamentenspenders befindet, wobei der erste und zweite Eingreifer mit dem Medikamentenbehälter in Eingriff bringbar sind.

4. Medikamentenspender nach Anspruch 2, bei dem die Anpassungseinrichtung eine Kupplung und einen Antriebsplattenmechanismus (1340; 1341) umfasst, der sich mit einer Gleitwelle (1311) verbindet, die den Medikamentenbehälter (1320) aufnimmt.

5. Medikamentenspender nach Anspruch 2, bei dem die Anpassungseinrichtung einen Zahnstangen- und Radmechanismus (1414; 1421) umfasst.

6. Medikamentenspender nach Anspruch 2, bei dem die Anpassungseinrichtung einen flexiblen Beutel umfasst, der ein viskoses Fluid enthält, wobei er sich geeigneterweise in der Basis des Gehäuses befindet.

7. Medikamentenspender nach Anspruch 2, bei dem die Anpassungseinrichtung einen Antriebskeilmechanismus (1510; 1520; 1530) umfasst.

8. Medikamentenspender nach einem der Ansprüche 1 bis 7, bei dem der Abgabemechanismus aus der Gruppe ausgewählt wird, die aus einem Ventil, einer Pumpe oder einem Kolbenmechanismus besteht.

9. Medikamentenspender nach einem der vorhergehenden Ansprüche, bei dem die Kopplung eine Formgedächtnislegierung umfasst.

10. Medikamentenspender nach Anspruch 9, bei dem die Formgedächtnislegierung eine Nickel-Titan-Legierung ist.

11. Medikamentenspender nach einem der Ansprüche 1 bis 10, bei dem der Medikamentenbehälter ein Aerosol-Behälter ist.

## Revendications

1. Distributeur de médicaments comprenant un logement ; un réservoir (1320 ; 1411) de médicaments comportant un mécanisme de distribution ; un siège (1330) de réservoir pour la réception du réservoir ; un dispositif d'ancrage sur le logement ou relié à celui-ci ; et un moyen (1350 ; 1422 ; 1540) d'entraînement pouvant déplacer le siège de réservoir par rapport au dispositif d'ancrage, afin d'actionner le mécanisme de distribution, le moyen d'entraînement comprenant un accouplement (1350 ; 1422 ; 1540) qui se déforme ou subit une transition de phase, en réponse à l'application d'une énergie thermique, d'une énergie de courant électrique, d'une énergie de champ électrique ou d'une énergie de champ magnétique, le distributeur de médicaments comprenant en outre, un moyen (1310, 1311, 1340, 1341 ; 1414, 1421 ; 1510, 1520, 1530) de réglage destiné à permettre à des réservoirs de médicaments de tailles variables d'être reçus par le logement.

2. Distributeur de médicaments selon la revendication 1, dans lequel le moyen de réglage comprend un moyen d'accouplement variable.

3. Distributeur de médicaments selon la revendication 2, dans lequel le moyen d'accouplement variable comprend un premier dispositif d'accouplement placé sur une première partie du distributeur de médicaments et un deuxième dispositif d'accouplement placé sur une deuxième partie du distributeur de médicaments, les premier et deuxième dispositifs d'accouplement pouvant coopérer avec le réservoir de médicaments.

4. Distributeur de médicaments selon la revendication 2, dans lequel le moyen de réglage comprend un mécanisme (1340, 1341) à embrayage et plateau d'entraînement qui se raccorde à un axe (1311) de chariot qui loge le réservoir (1320) de médicaments.

5. Distributeur de médicaments selon la revendication 2, dans lequel le moyen de réglage comprend un mécanisme (1414, 1421) à crémaillère et roue.

6. Distributeur de médicaments selon la revendication 2, dans lequel le moyen de réglage comprend un sac flexible contenant un fluide visqueux, positionné de manière appropriée, dans la base du logement.

7. Distributeur de médicaments selon la revendication 2, dans lequel le moyen de réglage comprend un mécanisme (1510, 1520, 1530) à pointe d'entraînement.

8. Distributeur de médicaments selon l'une quelconque des revendications 1 à 7, dans lequel le mécanisme de distribution est sélectionné dans le groupe composé d'un mécanisme de soupape, d'un mécanisme de pompe ou d'un mécanisme de piston plongeur.

9. Distributeur de médicaments selon l'une quelconque des revendications précédentes, dans lequel l'accouplement comprend un alliage à mémoire de forme.

10. Distributeur de médicaments selon la revendication 9, dans lequel l'alliage à mémoire de forme est un alliage de nickel-titane.

11. Distributeur de médicaments selon l'une quelconque des revendications 1 à 10, dans lequel le réservoir de médicaments est un contenant aérosol.
